# EUROPEAN PATENT APPLICATION

(11) **EP 2 623 509 A1**
(43) Date of publication of application: **07.08.2013**
(21) Application number: 13153396.0
(22) Date of filing: 31.01.2013
(51) Int. Cl.: C07F 15/00, C07C 209/52, C07F 17/02, C07C 211/48, C07C 213/08, C07C 217/84

(54) **Method of producing an optically active amine compound by catalytic asymmetric hydrogenation using a ruthenium-diphosphine complex**

(30) Priority: 01.02.2012 JP 2012019742
(71) Applicant: Kanto Kagaku Kabushiki Kaisha, Chuo-ku Tokyo (JP); National University Corporation Hokkaido University, Sapporo-shi, Hokkaido 060-0808 (JP)
(72) Inventor: Ohkuma, Takeshi, Hokkaido, Hokkaido 060-0808 (JP); Arai, Noriyoshi, Hokkaido, Hokkaido 060-0808 (JP); Utsumi, Noriyuki, Saitama, Saitama (JP); Murata, Kunihiko, Saitama, Saitama (JP); Tsutsumi, Kunihiko, Saitama, Saitama (JP)
(74) Representative: Zech, Stefan Markus

(57) **Abstract**

The present invention relates to a method for hydrogenating an imine to an optically active amine using a ruthenium metal complex represented by Formula (1)

RuXYAB (1)

such as RuBr₂[(*S*,*S* )-xylskewphos][(*S*,*S* )-dpen];
A is defined as a diphosphine compound represented by Formula (2), 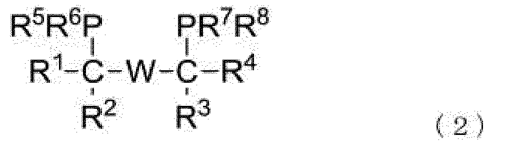 B is defined as a diamine compound represented by Formula (3)

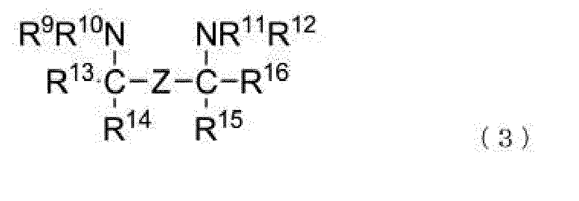

## Description

### [Technical Field]

The present invention relates to a method for producing an optically active amine compound using a ruthenium metal complex, and the like as a catalyst.

### [Background Art]

A method for producing an optically active amine compound is known in which a racemic amine compound is subjected to optical resolution using an optically active carboxylic acid. However, this method has the problem that the other optical isomer, which is not the target, remains. Furthermore, a method involving derivation from a natural optically active amine compound is also known, but this is not an easy method since it is difficult to obtain a desired starting material.

There have been many reports of methods for producing an optically active amine compound by means of catalytic asymmetric hydrogenation of an imine compound. For example, Non-Patent Document 1 describes a method for asymmetric hydrogenation of an imine using a chiral titanocene complex as a catalyst. Since this system requires the catalyst to be activated by adding butyllithium and phenylsilane, the range of applications is limited, and the catalytic activity is also low.

Non-Patent Document 2 describes a method for asymmetric hydrogenation of an imine compound using as a catalyst an iridium complex having a chiral monophosphine compound as a ligand. Furthermore, Non-Patent Document 3 described a method using as a catalyst an iridium complex having as a ligand f-BINAPHANE (1,1'-Bis[(R)-4,5-dihydro-3H-binaphtho[2,1-c:1',2'-e]phosphepino]ferrocene), which is a diphosphine compound. However, in these methods only an S/C (substrate/catalyst ratio) of up to 100 has been reported, the catalytic activity being low.

Patent Document 1 describes a method for producing an optically active amine compound by hydrogenating an aromatic imine compound using as a catalyst a ruthenium-Cl₂ complex having as ligands a diamine compound and a diphosphine compound such as BINAP (2,2'-bis(diphenylphosphino)-1,1'-binaphthyl) or Me-DuPHOS (1,2-bis(2,5-dimethylphospholano)benzene). However, the catalytic activity is low, and the enantioselectivity of the reaction is not sufficient. Patent Document 2 describes a method for producing an amine compound by hydrogenating a dialkylimine compound, and the like using as a catalyst a ruthenium-HCl complex having the same type of diphosphine compound as above and a diamine compound as ligands. Although the catalytic activity is improved, it is not yet sufficient and there is no mention of the enantioselectivity. Moreover, the ruthenium-HCl complex is unstable and difficult to handle.

On the other hand, Patent Document 3 describes a method for producing an alcohol compound by hydrogenating a ketone compound using as a catalyst a ruthenium-Br₂ complex having a diamine compound and a 1,3-diphosphine compound such as XylSKEWPHOS (2,4-bis(di-3,5-xylylphosphino)pentane) as ligands. Furthermore, Patent Document 4 describes a method for producing an alcohol compound by hydrogenating a ketone compound using as a catalyst a ruthenium-H(BH₄) complex having a diamine compound and a 1,3-diphosphine compound such as XylSKEWPHOS (2,4-bis(di-3,5-xylylphosphino)pentane) as ligands. However, these documents do not mention hydrogenation of an imine compound at all.

Therefore, there has been a desire for a method for producing an optically active amine compound by hydrogenating an imine compound with high enantioselectivity using a metal complex having high catalytic activity.
As hereinbefore described, the catalytic activity of the conventional hydrogenation catalysts is low, and the enantioselectivity thereof is not sufficient either.

### [Prior Art Documents]

### [Patent Documents]

[Patent Document 1] International Patent Publication WO 02/08169 A1
[Patent Document 2] International Patent Publication WO 03/097571 A1
[Patent Document 3] Japanese Patent No. 3566955
[Patent Document 4] JP, A, 2004-238306

### [Non-Patent Documents]

[Non-Patent Document 1] J. Am. Chem. Soc., 1992, 114, 7562-7564
[Non-Patent Document 2] J. Am. Chem. Soc., 2009, 131, 8358-8359
[Non-Patent Document 3] Angew. Chem. Int. Ed. 2001, 40, 3425-3428

### [Summary of the Invention]

### [Problems to be Solved by the Invention]

The present invention has been accomplished in order to solve the above-mentioned problems, and it is an object thereof to provide a method for producing an optically active amine compound by means of a highly enantioselective hydrogenation reaction of an imine compound using a ruthenium metal complex having high catalytic activity.

### [Means for Solving the Problems]

As a result of an intensive investigation by the present inventors in order to solve the above-mentioned problems, it has been found that a ruthenium complex having a specific diphosphine compound as a ligand exhibits excellent catalytic activity and enantioselectivity in an asymmetric hydrogenation reaction of an imine compound in the presence of an aprotic solvent or under solvent-free conditions, and the present invention has thus been accomplished.

The present invention relates to the following.
[1] A method for producing an optically active amine compound represented by Formula (5) 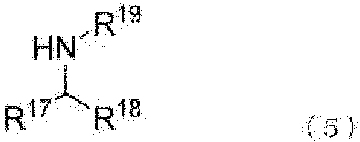 (R¹⁷ and R¹⁸ are independently selected from the group consisting of optionally substituted alkyl, optionally substituted alkenyl, optionally substituted alkynyl, optionally substituted cycloalkyl, optionally substituted cycloalkenyl, optionally substituted aryl, optionally substituted heteroaryl, optionally substituted aralkyl, and optionally substituted heteroaralkyl,
   R¹⁹ is hydrogen, optionally substituted alkyl, optionally substituted alkenyl, optionally substituted alkynyl, optionally substituted cycloalkyl, optionally substituted cycloalkenyl, optionally substituted aryl, optionally substituted heteroaryl, optionally substituted aralkyl, or optionally substituted heteroaralkyl, and
   two of R¹⁷, R¹⁸, and R¹⁹ may together form an optionally substituted ring),
   in which a ruthenium complex represented by Formula (1)

   RuXYAB (1)

   (X and Y are each independently hydrogen or an anionic group, A is a diphosphine compound represented by Formula (2), 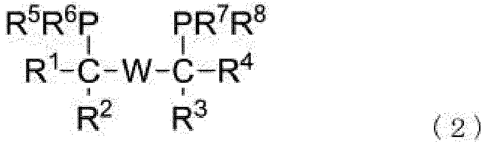 (R¹, R², R³, and R⁴ are each independently hydrogen or an optionally substituted chain-form or cyclic hydrocarbon group having 1 to 20 carbons,
   R⁵, R⁶, R⁷, and R⁸ are each independently an optionally substituted hydrocarbon group having 1 to 30 carbons, and W is an optionally substituted hydrocarbon group having 1 or 2 carbons, or a single bond), and
   B is a diamine compound represented by Formula (3) 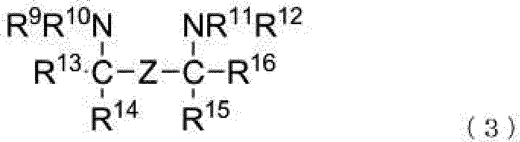 (R⁹, R¹⁰, R¹¹, and R¹² are each independently hydrogen or an optionally substituted chain-form or cyclic hydrocarbon group having 1 to 30 carbons, wherein at least one of R⁹, R¹⁰, R¹¹, and R¹² is hydrogen,
   R¹³, R¹⁴, R¹⁵, and R¹⁶ are each independently hydrogen or an optionally substituted chain-form or cyclic hydrocarbon group having 1 to 30 carbons, and
   Z is an optionally substituted chain-form or cyclic hydrocarbon group having 1 to 10 carbons, or a single bond), each of the ligands of ruthenium being able to coordinate in any way), an imine compound represented by Formula (4) 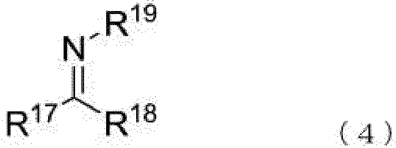 (R¹⁷, R¹⁸, and R¹⁹ are as defined above), and
   one or more bases selected from the group consisting of an alkali metal or alkaline earth metal salt, a quaternary ammonium salt, and a tertiary amine are mixed in an aprotic solvent or without a solvent under pressurized hydrogen to thus hydrogenate the imine compound.

[2] A method for producing an optically active amine compound represented by Formula (5) 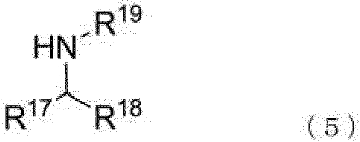 (R¹⁷, R¹⁸, and R¹⁹ are as defined in [1]),
   in which a ruthenium complex represented by Formula (6) RuXYALₙ (6)
   (L is an organic ligand, n is any number between 0 and 2, and X, Y, and A are as defined in [1]),
   a diamine compound represented by Formula (3) 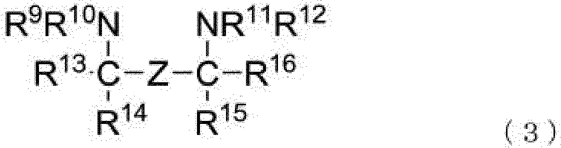 (R⁹ to R¹⁶ are as defined in [1]),
   an imine compound represented by Formula (4) 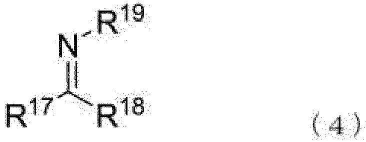 (R¹⁷, R¹⁸, and R¹⁹ are as defined in [1]), and
   one or more bases selected from the group consisting of an alkali metal or alkaline earth metal salt, a quaternary ammonium salt, and a tertiary amine are mixed in an aprotic solvent or without a solvent under pressurized hydrogen to thus hydrogenate the imine compound.

[3] The method according to [1] or [2], wherein in Formula (1) or (6), A is SKEWPHOS: 2,4-bis(diphenylphosphino)pentane, TolSKEWPHOS: 2,4-bis(di-4-tolylphosphino)pentane, XylSKEWPHOS: 2,4-bis(di-3,5-xylylphosphino)pentane, or ModSKEWPHOS: 2,4-bis[bis(3,5-dimethyl-4-methoxyphenyl)phosphino]pentane.

[4] The method according to any one of [1] to [3], wherein in Formula (3), R⁹, R¹⁰, R¹¹, R¹², R¹³, and R¹⁵ are hydrogen; R¹⁴ is hydrogen, a phenyl group, a 4-trifluoromethylphenyl group, a 4-fluorophenyl group, a 4-methoxyphenyl group, or a 3-methoxyphenyl group; R¹⁶ is a phenyl group, a 4-trifluoromethylphenyl group, a 4-fluorophenyl group, a 4-methoxyphenyl group, or a 3-methoxyphenyl group; and Z is a single bond.

[5] The method according to any one of [1] to [4], wherein the aprotic solvent is one or more selected from the group consisting of toluene, benzene, tetrahydrofuran, and *tert*-butyl methyl ether.

[6] The method according to any one of [1] to [5], wherein in Formula (4), R¹⁷ is optionally substituted alkenyl, optionally substituted alkynyl, optionally substituted cycloalkenyl, optionally substituted aryl, or optionally substituted heteroaryl; R¹⁸ is different from R¹⁷ and is optionally substituted alkyl, optionally substituted cycloalkyl, optionally substituted alkenyl, optionally substituted cycloalkenyl, or optionally substituted aryl; R¹⁷ and R¹⁸ may together form an optionally substituted ring; and R¹⁹ is optionally substituted alkyl, optionally substituted alkenyl, optionally substituted alkynyl, optionally substituted aryl, optionally substituted heteroaryl, or optionally substituted aralkyl.

[7] The method according to any one of [1] to [5], wherein in Formula (4), R¹⁷ is optionally substituted alkenyl, optionally substituted aryl, or optionally substituted heteroaryl; R¹⁸ is optionally substituted alkyl or optionally substituted cycloalkyl; R¹⁷ and R¹⁸ may together form an optionally substituted ring; and R¹⁹ is optionally substituted alkyl, optionally substituted aryl, or optionally substituted aralkyl.

In accordance with the above-mentioned constitution, the present invention enables an optically active amine compound having high optical purity to be produced by efficiently hydrogenating an imine compound. The optically active amine compound thus obtained does not require a complicated and expensive purification process, and the like for optical resolution, and may be used directly in the synthesis of for example a pharmaceutical compound, an agrochemical compound, an intermediate therefor, and the like.

Although the mechanism by which the above-mentioned effect is accomplished is not clear, the present inventors have confirmed that in the method of the present invention the hydrogenation reaction proceeds more efficiently in the presence of an aprotic solvent or under solvent-free conditions, and the hydrogenation reaction hardly proceeds at all in the presence of a protic solvent such as an alcohol. It is therefore surmised that, in the production methods of the present invention, hydrogenating an imine compound in the presence of an aprotic solvent or under solvent-free conditions is essential for giving a reaction mechanism for obtaining an optically active amine in high yield and high optical purity.

### [Modes for Carrying Out the Invention]

The present invention is explained in detail below.
A ruthenium complex used in the method of the present invention is not particularly limited as long as it enables an imine compound to be hydrogenated to an optically active amine compound, but is typically represented by Formula (1).

RuXYAB (1)

X and Y in Formula (1) are each independently, without particular limitation, for example hydrogen or an anionic group.
Examples of X and Y above include hydrogen, a fluorine atom, a chlorine atom, a bromine atom, an iodine atom, an acetoxy group, a benzoyloxy group, a (2,6-dihydroxybenzoyl)oxy group, a (2,5-dihydroxybenzoyl)oxy group, a (3-aminobenzoyl)oxy group, a (2,6-methoxybenzoyl)oxy group, a (2,4,6-triisopropylbenzoyl)oxy group, a 1-naphthalenecarbonyloxy group, a 2-naphthalenecarbonyloxy group, a trifluoroacetoxy group, a trifluoromethanesulfoxy group, a tetrahydroborate anion, a tetrafluoroborate anion, and a tetrakis[3,5-bis(trifluoromethyl)phenyl]borate anion. From the viewpoint of catalytic activity and complex stability, they are preferably hydrogen, a halogen atom such as a fluorine atom, a chlorine atom, a bromine atom, or an iodine atom, a tetrahydroborate anion, or a tetrafluoroborate anion, and particularly preferably hydrogen, a chlorine atom, a bromine atom, an iodine atom, or a tetrahydroborate anion.
With regard to combination of X and Y, from the viewpoint of catalytic activity and complex stability, it is preferable that X is one selected from the group consisting of hydrogen, a chlorine atom, a bromine atom, and an iodine atom and Y is one selected from the group consisting of a chlorine atom, a bromine atom, an iodine atom, and a tetrahydroborate anion.

Diphosphine compound A in Formula (1) is represented by Formula (2).

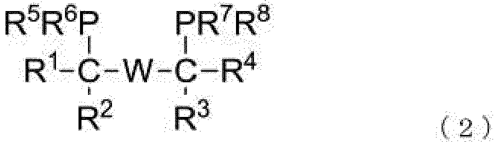

R¹, R², R³, and R⁴ are each independently, without particular limitation, for example hydrogen, an aliphatic or alicyclic saturated or unsaturated hydrocarbon group, or a monocyclic or polycyclic aromatic or araliphatic hydrocarbon group, the hydrocarbon group optionally further having one or more substituents.

Examples of R¹, R², R³, and R⁴ above include hydrogen and a hydrocarbon group such as an alkyl group, an alkenyl group, an alkynyl group, a cycloalkyl group, a cycloalkenyl group, an aryl group, and an aralkyl group. From the viewpoint of catalytic activity and enantioselectivity, they are preferably hydrogen or an optionally substituted chain-form or cyclic hydrocarbon group having 1 to 20 carbons, more preferably hydrogen, a methyl group, an ethyl group, a propyl group, a phenyl group or a substituted phenyl group, and particularly preferably hydrogen, a methyl group, or a phenyl group.

With regard to combination of R¹, R², R³, and R⁴, from the viewpoint of catalytic activity and enantioselectivity, it is preferable that R¹ and R³ are hydrogen and R² and R⁴ are a methyl group or a phenyl group.
The hydrocarbon group optionally has further various types of acceptable substituents such as an alkyl group, an alkenyl group, an alkynyl group, a cycloalkyl group, a cycloalkenyl group, an aryl group, a heteroaryl group, an alkoxy group, an ester group, an acyloxy group, a halogen atom, a nitro group, a cyano group, or a perfluoroalkyl group.

R ⁵ R⁶, R⁷, and R⁸ are each independently, without particular limitation, for example an aliphatic or alicyclic saturated or unsaturated hydrocarbon group or a monocyclic or polycyclic aromatic or araliphatic hydrocarbon group, and the hydrocarbon group optionally further has one or more substituents.

Examples of R⁵, R⁶, R⁷, and R⁸ above include a hydrocarbon group such as an alkyl group, an alkenyl group, an alkynyl group, a cycloalkyl group, a cycloalkenyl group, an aryl group, or an aralkyl group. From the viewpoint of catalytic activity and enantioselectivity, they are preferably an optionally substituted hydrocarbon group having 1 to 30 carbons, more preferably a phenyl group or a substituted phenyl group, and particularly preferably a phenyl group or a phenyl group having one to five methyl groups, ethyl groups, propyl groups, *tert-*butyl groups, or methoxy groups as substituents.
With regard to combination of R⁵, R⁶, R⁷, and R⁸, from the viewpoint of catalytic activity and enantioselectivity, it is preferable that all of R⁵, R⁶, R⁷, and R⁸ are a phenyl group or a phenyl group having one or more substituents, and more preferably a phenyl group or a phenyl group having an alkyl group or an alkoxy group.

With regard to combination of R¹, R², R³, R⁴, R⁵, R⁶, R⁷, and R⁸, from the viewpoint of catalytic activity and enantioselectivity, it is preferable that R¹ and R³ are hydrogen, R² and R⁴ are a methyl group or a phenyl group, and all of R⁵, R⁶, R⁷, and R⁸ are a phenyl group or a phenyl group having an alkyl group or an alkoxy group.

The hydrocarbon group optionally further has various types of acceptable substituents such as an alkyl group, an alkenyl group, an alkynyl group, a cycloalkyl group, a cycloalkenyl group, an aryl group, a heteroaryl group, an alkoxy group, an ester group, an acyloxy group, a halogen atom, a nitro group, a cyano group, or a perfluoroalkyl group.

Examples of the alkyl group include a methyl group, an ethyl group, an *n*-propyl group, an isopropyl group, an *n*-butyl group, a *sec-*butyl group, an isobutyl group, and a *tert*-butyl group.
Examples of the alkenyl group include a vinyl group, a 1-propenyl group, an allyl group, an isopropenyl group, a 1-butenyl group, a crotyl group, a 3-butenyl group, an isobutenyl group, a methallyl group, and a 2-phenylvinyl group.
Examples of the alkynyl group include an ethynyl group, a 1-propynyl group, a propargyl group, a 1-butynyl group, a 2-butynyl group, a 3-butynyl group, a 3-methyl-1-butynyl group, and a 2-phenylethynyl group.

Examples of the cycloalkyl group include a cyclopropyl group, a cyclobutyl group, a cyclopentyl group, and a cyclohexyl group.
Examples of the cycloalkenyl group include a cyclopropenyl group, a cyclobutenyl group, a cyclopentenyl group, and a cyclohexenyl group.
Examples of the aryl group include a phenyl group, a tolyl group, a xylyl group, a ferrocenyl group, and a naphthyl group.

Examples of the aralkyl group include a benzyl group, a phenethyl group, and a phenylpropyl group.
Examples of the heteroaryl group include a pyridyl group, a pyrimidyl group, a pyrazyl group, a quinolyl group, an isoquinolyl group, a thienyl group, a furyl group, a pyrrolyl group, an indolyl group, an imidazolyl group, a pyrazolyl group, a thiazolyl group, and a triazolyl group.

Examples of the alkoxy group include a methoxy group, an ethoxy group, a propoxy group, and a butoxy group.
Examples of the ester group include a methoxycarbonyl group, an ethoxycarbonyl group, a butoxycarbonyl group, and a benzyloxycarbonyl group.
Examples of the acyloxy group include an acetoxy group, a trifluoroacetoxy group, an acryloxy group, and a methacryloxy group.
Examples of the halogen atom include a fluorine atom, a chlorine atom, a bromine atom, and an iodine atom.
Examples of the perfluoroalkyl group include a trifluoromethyl group and a pentafluoroethyl group.

Examples of W include, without particular limitation, an optionally substituted hydrocarbon group having 1 or 2 carbons, and a single bond.
Examples of the optionally substituted hydrocarbon group having 1 or 2 carbons include a methylene group, an ethylene group, and a vinylene group. From the viewpoint of catalytic activity and enantioselectivity, it is preferably an unsubstituted methylene group.

Specific examples of the diphosphine compound A represented by Formula (2) are listed.
Examples of pentane derivatives having a diphenylphosphino group at the 2-position and the 4-position include SKEWPHOS: 2,4-bis(diphenylphosphino)pentane, 2,4-bis(diphenylphosphino)-3-methylpentane, 2,4-bis(diphenylphosphino)-3,3-dimethylpentane, 2,4-bis(diphenylphosphino)-3-ethylpentane, 2,4-bis(diphenylphosphino)-3,3-diethylpentane, 2,4-bis(diphenylphosphino)-3-propylpentane, 2,4-bis(diphenylphosphino)-3,3-dipropylpentane, 2,4-bis(diphenylphosphino)-3-isopropylpentane, 2,4-bis(diphenylphosphino)-3,3-diisopropylpentane, 2,4-bis(diphenylphosphino)-3-ethyl-3-methylpentane, 2,4-bis(diphenylphosphino)-3-methyl-3-propylpentane, 2,4-bis(diphenylphosphino)-3-methyl-3-isopropylpentane, 2,4-bis(diphenylphosphino)-3-ethyl-3-propylpentane, 2,4-bis(diphenylphosphino)-3-ethyl-3-isopropylpentane, and 2,4-bis(diphenylphosphino)-3-propyl-3-isopropylpentane.

Examples of pentane derivatives having a di-4-tolylphosphino group at the 2-position and the 4-position include TolSKEWPHOS: 2,4-bis(di-4-tolylphosphino)pentane, 2,4-bis(di-4-tolylphosphino)-3-methylpentane, 2,4-bis(di-4-tolylphosphino)-3,3-dimethylpentane, 2,4-bis(di-4-tolylphosphino)-3-ethylpentane, 2,4-bis(di-4-tolylphosphino)-3,3-diethylpentane, 2,4-bis(di-4-tolylphosphino)-3-propylpentane, 2,4-bis(di-4-tolylphosphino)-3,3-dipropylpentane, 2,4-bis(di-4-tolylphosphino)-3-isopropylpentane, 2,4-bis(di-4-tolylphosphino)-3,3-diisopropylpentane, 2,4-bis(di-4-tolylphosphino)-3-ethyl-3-methylpentane, 2,4-bis(di-4-tolylphosphino)-3-methyl-3-propylpentane, 2,4-bis(di-4-tolylphosphino)-3-methyl-3-isopropylpentane, 2,4-bis(di-4-tolylphosphino)-3-ethyl-3-propylpentane, 2,4-bis(di-4-tolylphosphino)-3-ethyl-3-isopropylpentane, and 2,4-bis(di-4-tolylphosphino)-3-propyl-3-isopropylpentane.

Examples of pentane derivatives having a di(4-*tert-*butylphenyl)phosphino group at the 2-position and the 4-position include 4-*t*-BuSKEWPHOS: 2,4-bis[di(4-*tert-*butylphenyl)phosphino]pentane, 2,4-bis[di(4-*tert-*butylphenyl)phosphino]-3-methylpentane, 2,4-bis[di(4-*tert-*butylphenyl)phosphino]-3,3-dimethylpentane, 2,4-bis[di(4-*tert-*butylphenyl)phosphino]-3-ethylpentane, 2,4-bis[di(4-*tert-*butylphenyl)phosphino]-3,3-diethylpentane, 2,4-bis[di(4-*tert-*butylphenyl)phosphino]-3-propylpentane, 2,4-bis[di(4-*tert-*butylphenyl)phosphino]-3,3-dipropylpentane, 2,4-bis[di(4-*tert-*butylphenyl)phosphino]-3-isopropylpentane, 2,4-bis[di(4-*tert-*butylphenyl)phosphino]-3,3-diisopropylpentane, 2,4-bis[di(4-*tert-*butylphenyl)phosphino]-3-ethyl-3-methylpentane, 2,4-bis[di(4-tert-butylphenyl)phosphino]-3-methyl-3-propylpentane, 2,4-bis[di(4-*tert*-butylphenyl)phosphino]-3-methyl-3-isopropylpentane, 2,4-bis[di(4-*tert*-butylphenyl)phosphino]-3-ethyl-3-propylpentane, 2,4-bis[di(4-*tert*-butylphenyl)phosphino]-3-ethyl-3-isopropylpentane, and 2,4-bis[di(4-*tert*-butylphenyl)phosphino]-3-propyl-3-isopropylpentane.

Examples of pentane derivatives having a di-3,5-xylylphosphino group at the 2-position and the 4-position include XylSKEWPHOS: 2,4-bis(di-3,5-xylylphosphino)pentane, 2,4-bis(di-3,5-xylylphosphino)-3-methylpentane, 2,4-bis(di-3,5-xylylphosphino)-3,3-dimethylpentane, 2,4-bis(di-3,5-xylylphosphino)-3-ethylpentane, 2,4-bis(di-3,5-xylylphosphino)-3,3-diethylpentane, 2,4-bis(di-3,5-xylylphosphino)-3-propylpentane, 2,4-bis(di-3,5-xylylphosphino)-3,3-dipropylpentane, 2,4-bis(di-3,5-xylylphosphino)-3-isopropylpentane, 2,4-bis(di-3,5-xylylphosphino)-3,3-diisopropylpentane, 2,4-bis(di-3,5-xylylphosphino)-3-ethyl-3-methylpentane, 2,4-bis(di-3,5-xylylphosphino)-3-methyl-3-propylpentane, 2,4-bis(di-3,5-xylylphosphino)-3-methyl-3-isopropylpentane, 2,4-bis(di-3,5-xylylphosphino)-3-ethyl-3-propylpentane, 2,4-bis(di-3,5-xylylphosphino)-3-ethyl-3-isopropylpentane, and 2,4-bis(di-3,5-xylylphosphino)-3-propyl-3-isopropylpentane.

Examples of pentane derivatives having a bis(3,5-diethylphenyl)phosphino group at the 2-position and the 4-position include 3,5-diEtSKEWPHOS: 2,4-bis[bis(3,5-diethylphenyl)phosphino]pentane, 2,4-bis[bis(3,5-diethylphenyl)phosphino]-3-methylpentane, 2,4-bis[bis(3,5-diethylphenyl)phosphino]-3,3-dimethylpentane, 2,4-bis[bis(3,5-diethylphenyl)phosphino]-3-ethylpentane, 2,4-bis[bis(3,5-diethylphenyl)phosphino]-3,3-diethylpentane, 2,4-bis[bis(3,5-diethylphenyl)phosphino]-3-propylpentane, 2,4-bis[bis(3,5-diethylphenyl)phosphino]-3,3-dipropylpentane, 2,4-bis[bis(3,5-diethylphenyl)phosphino]-3-isopropylpentane, 2,4-bis[bis(3,5-diethylphenyl)phosphino]-3,3-diisopropylpentane, 2,4-bis[bis(3,5-diethylphenyl)phosphino]-3-ethyl-3-methylpentane, 2,4-bis[bis(3,5-diethylphenyl)phosphino]-3-methyl-3-propylpentane, 2,4-bis[bis(3,5-diethylphenyl)phosphino]-3-methyl-3-isopropylpentane, 2,4-bis[bis(3,5-diethylphenyl)phosphino]-3-ethyl-3-propylpentane, 2,4-bis[bis(3,5-diethylphenyl)phosphino]-3-ethyl-3-isopropylpentane, and 2,4-bis[bis(3,5-diethylphenyl)phosphino]-3-propyl-3-isopropylpentane.

Examples of pentane derivatives having a bis(3,5-dimethyl-4-methoxyphenyl)phosphino group at the 2-position and the 4-position include ModSKEWPHOS: 2,4-bis[bis(3,5-dimethyl-4-methoxyphenyl)phosphino]pentane, 2,4-bis[bis(3,5-dimethyl-4-methoxyphenyl)phosphino]-3-methylpentane, 2,4-bis[bis(3,5-dimethyl-4-methoxyphenyl)phosphino]-3,3-dimethylpentane, 2,4-bis[bis(3,5-dimethyl-4-methoxyphenyl)phosphino]-3-ethylpentane, 2,4-bis[bis(3,5-dimethyl-4-methoxyphenyl)phosphino]-3,3-diethylpentane, 2,4-bis[bis(3,5-dimethyl-4-methoxyphenyl)phosphino]-3-propylpentane, 2,4-bis[bis(3,5-dimethyl-4-methoxyphenyl)phosphino]-3,3-dipropylpentane, 2,4-bis[bis(3,5-dimethyl-4-methoxyphenyl)phosphino]-3-isopropylpentane, 2,4-bis[bis(3,5-dimethyl-4-methoxyphenyl)phosphino]-3,3-diisopropylpentane, 2,4-bis[bis(3,5-dimethyl-4-methoxyphenyl)phosphino]-3-ethyl-3-methylpentane, 2,4-bis[bis(3,5-dimethyl-4-methoxyphenyl)phosphino]-3-methyl-3-propylpentane, 2,4-bis[bis(3,5-dimethyl-4-methoxyphenyl)phosphino]-3-methyl-3-isopropylpentane, 2,4-bis[bis(3,5-dimethyl-4-methoxyphenyl)phosphino]-3-ethyl-3-propylpentane, 2,4-bis[bis(3,5-dimethyl-4-methoxyphenyl)phosphino]-3-ethyl-3-isopropylpentane, and 2,4-bis[bis(3,5-dimethyl-4-methoxyphenyl)phosphino]-3-propyl-3-isopropylpentane.

Examples of 1,3-diphenylpropane derivatives having a diphenylphosphino group at the 1-position and the 3-position include 1,3-bis(diphenylphosphino)-1,3-diphenylpropane, 1,3-bis(diphenylphosphino)-1,3-diphenyl-2-methylpropane, 1,3-bis(diphenylphosphino)-1,3-diphenyl-2,2-dimethylpropane, 1,3-bis(diphenylphosphino)-1,3-diphenyl-2-ethylpropane, 1,3-bis(diphenylphosphino)-1,3-diphenyl-2,2-diethylpropane, 1,3-bis(diphenylphosphino)-1,3-diphenyl-2-propylpropane, 1,3-bis(diphenylphosphino)-1,3-diphenyl-2,2-dipropylpropane, 1,3-bis(diphenylphosphino)-1,3-diphenyl-2-isopropylpropane, 1,3-bis(diphenylphosphino)-1,3-diphenyl-2,2-diisopropylpropane, 1,3-bis(diphenylphosphino)-1,3-diphenyl-2-ethyl-2-methylpropane, 1,3-bis(diphenylphosphino)-1,3-diphenyl-2-methyl-2-propylpropane, 1,3-bis(diphenylphosphino)-1,3-diphenyl-2-methyl-2-isopropylpropane, 1,3-bis(diphenylphosphino)-1,3-diphenyl-2-ethyl-2-propylpropane, 1,3-bis(diphenylphosphino)-1,3-diphenyl-2-ethyl-2-isopropylpropane, and 1,3-bis(diphenylphosphino)-1,3-diphenyl-2-propyl-2-isopropylpropane.

Examples of 1,3-diphenylpropane derivatives having a di-4-tolylphosphino group at the 1-position and the 3-position include 1,3-bis(di-4-tolylphosphino)-1,3-diphenylpropane, 1,3-bis(di-4-tolylphosphino)-1,3-diphenyl-2-methylpropane, 1,3-bis(di-4-tolylphosphino)-1,3-diphenyl-2,2-dimethylpropane, 1,3-bis(di-4-tolylphosphino)-1,3-diphenyl-2-ethylpropane, 1,3-bis(di-4-tolylphosphino)-1,3-diphenyl-2,2-diethylpropane, 1,3-bis(di-4-tolylphosphino)-1,3-diphenyl-2-propylpropane, 1,3-bis(di-4-tolylphosphino)-1,3-diphenyl-2,2-dipropylpropane, 1,3-bis(di-4-tolylphosphino)-1,3-diphenyl-2-isopropylpropane, 1,3-bis(di-4-tolylphosphino)-1,3-diphenyl-2,2-diisopropylpropane, 1,3-bis(di-4-tolylphosphino)-1,3-diphenyl-2-ethyl-2-methylpropane, 1,3-bis(di-4-tolylphosphino)-1,3-diphenyl-2-methyl-2-propylpropane, 1,3-bis(di-4-tolylphosphino)-1,3-diphenyl-2-methyl-2-isopropylpropane, 1,3-bis(di-4-tolylphosphino)-1,3-diphenyl-2-ethyl-2-propylpropane, 1,3-bis(di-4-tolylphosphino)-1,3-diphenyl-2-ethyl-2-isopropylpropane, and 1,3-bis(di-4-tolylphosphino)-1,3-diphenyl-2-propyl-2-isopropylpropane.

Examples of 1,3-diphenylpropane derivatives having a di(4-tert-butylphenyl)phosphino group at the 1-position and the 3-position include 1,3-bis[di(4-*tert*-butylphenyl)phosphino]-1,3-diphenylpropane, 1,3-bis[di(4-*tert*-butylphenyl)phosphino]-1,3-diphenyl-2-methylpropane, 1,3-bis[di(4-*tert-*butylphenyl)phosphino]-1,3-diphenyl-2,2-dimethylpropane, 1,3-bis[di(4-*tert*-butylphenyl)phosphino]-1,3-diphenyl-2-ethylpropane, 1,3-bis[di(4-*tert*-butylphenyl)phosphino]-1,3-diphenyl-2,2-diethylpropane, 1,3-bis[di(4-*tert*-butylphenyl)phosphino]-1,3-diphenyl-2-propylpropane, 1,3-bis[di(4-*tert-*butylphenyl)phosphino]-1,3-diphenyl-2,2-dipropylpropane, 1,3-bis[di(4-*tert*-butylphenyl)phosphino]-1,3-diphenyl-2-isopropylpropane, 1,3-bis[di(4-*tert*-butylphenyl)phosphino]-1,3-diphenyl-2,2-diisopropylpropane, 1,3-bis[di(4-*tert-*butylphenyl)phosphino]-1,3-diphenyl-2-ethyl-2-methylpropane, 1,3-bis[di(4-*tert*-butylphenyl)phosphino]-1,3-diphenyl-2-methyl-2-propylpropane, 1,3-bis[di(4-*tert*-butylphenyl)phosphino]-1,3-diphenyl-2-methyl-2-isopropylpropane, 1,3-bis[di(4-*tert-*butylphenyl)phosphino]-1,3-diphenyl-2-ethyl-2-propylpropane, 1,3-bis[di(4-*tert*-butylphenyl)phosphino]-1,3-diphenyl-2-ethyl-2-isopropylpropane, and 1,3-bis[di(4-*tert*-butylphenyl)phosphino]-1,3-diphenyl-2-propyl-2-isopropylpropane.

Examples of 1,3-diphenylpropane derivatives having a di-3,5-xylylphosphino group at the 1-position and the 3-position include 1,3-bis(di-3,5-xylylphosphino)-1,3-diphenylpropane, 1,3-bis(di-3,5-xylylphosphino)-1,3-diphenyl-2-methylpropane, 1,3-bis(di-3,5-xylylphosphino)-1,3-diphenyl-2,2-dimethylpropane, 1,3-bis(di-3,5-xylylphosphino)-1,3-diphenyl-2-ethylpropane, 1,3-bis(di-3,5-xylylphosphino)-1,3-diphenyl-2,2-diethylpropane, 1,3-bis(di-3,5-xylylphosphino)-1,3-diphenyl-2-propylpropane, 1,3-bis(di-3,5-xylylphosphino)-1,3-diphenyl-2,2-dipropylpropane, 1,3-bis(di-3,5-xylylphosphino)-1,3-diphenyl-2-isopropylpropane, 1,3-bis(di-3,5-xylylphosphino)-1,3-diphenyl-2,2-diisopropylpropane, 1,3-bis(di-3,5-xylylphosphino)-1,3-diphenyl-2-ethyl-2-methylpropane, 1,3-bis(di-3,5-xylylphosphino)-1,3-diphenyl-2-methyl-2-propylpropane, 1,3-bis(di-3,5-xylylphosphino)-1,3-diphenyl-2-methyl-2-isopropylpropane, 1,3-bis(di-3,5-xylylphosphino)-1,3-diphenyl-2-ethyl-2-propylpropane, 1,3-bis(di-3,5-xylylphosphino)-1,3-diphenyl-2-ethyl-2-isopropylpropane, and 1,3-bis(di-3,5-xylylphosphino)-1,3-diphenyl-2-propyl-2-isopropylpropane.

Examples of 1,3-diphenylpropane derivatives having a bis(3,5-diethylphenyl)phosphino group at the 1-position and the 3-position include 1,3-bis[bis(3,5-diethylphenyl)phosphino]-1,3-diphenylpropane, 1,3-bis[bis(3,5-diethylphenyl)phosphino]-1,3-diphenyl-2-methylpropane, 1,3-bis[bis(3,5-diethylphenyl)phosphino]-1,3-diphenyl-2,2-dimethylpropane, 1,3-bis[bis(3,5-diethylphenyl)phosphino]-1,3-diphenyl-2-ethylpropane, 1,3-bis[bis(3,5-diethylphenyl)phosphino]-1,3-diphenyl-2,2-diethylpropane, 1,3-bis[bis(3,5-diethylphenyl)phosphino]-1,3-diphenyl-2-propylpropane, 1,3-bis[bis(3,5-diethylphenyl)phosphino]-1,3-diphenyl-2,2-dipropylpropane, 1,3-bis[bis(3,5-diethylphenyl)phosphino]-1,3-diphenyl-2-isopropylpropane, 1,3-bis[bis(3,5-diethylphenyl)phosphino]-1,3-diphenyl-2,2-diisopropylpropane, 1,3-bis[bis(3,5-diethylphenyl)phosphino]-1,3-diphenyl-2-ethyl-2-methylpropane, 1,3-bis[bis(3,5-diethylphenyl)phosphino]-1,3-diphenyl-2-methyl-2-propylpropane, 1,3-bis[bis(3,5-diethylphenyl)phosphino]-1,3-diphenyl-2-methyl-2-isopropylpropane, 1,3-bis[bis(3,5-diethylphenyl)phosphino]-1,3-diphenyl-2-ethyl-2-propylpropane, 1,3-bis[bis(3,5-diethylphenyl)phosphino]-1,3-diphenyl-2-ethyl-2-isopropylpropane, and 1,3-bis[bis(3,5-diethylphenyl)phosphino]-1,3-diphenyl-2-propyl-2-isopropylpropane.

Examples of 1,3-diphenylpropane derivatives having a bis(3,5-dimethyl-4-methoxyphenyl)phosphino group at the 1-position and the 3-position include 1,3-bis[bis(3,5-dimethyl-4-methoxyphenyl)phosphino]-1,3-diphenylpropane, 1,3-bis[bis(3,5-dimethyl-4-methoxyphenyl)phosphino]-1,3-diphenyl-2-methylpropane, 1,3-bis[bis(3,5-dimethyl-4-methoxyphenyl)phosphino]-1,3-diphenyl-2,2-dimethylpropane, 1,3-bis[bis(3,5-dimethyl-4-methoxyphenyl)phosphino]-1,3-diphenyl-2-ethylpropane, 1,3-bis[bis(3,5-dimethyl-4-methoxyphenyl)phosphino]-1,3-diphenyl-2,2-diethylpropane, 1,3-bis[bis(3,5-dimethyl-4-methoxyphenyl)phosphino]-1,3-diphenyl-2-propylpropane, 1,3-bis[bis(3,5-dimethyl-4-methoxyphenyl)phosphino]-1,3-diphenyl-2,2-dipropylpropane, 1,3-bis[bis(3,5-dimethyl-4-methoxyphenyl)phosphino]-1,3-diphenyl-2-isopropylpropane, 1,3-bis[bis(3,5-dimethyl-4-methoxyphenyl)phosphino]-1,3-diphenyl-2,2-diisopropylpropane, 1,3-bis[bis(3,5-dimethyl-4-methoxyphenyl)phosphino]-1,3-diphenyl-2-ethyl-2-methylpropane, 1,3-bis[bis(3,5-dimethyl-4-methoxyphenyl)phosphino]-1,3-diphenyl-2-methyl-2-propylpropane, 1,3-bis[bis(3,5-dimethyl-4-methoxyphenyl)phosphino]-1,3-diphenyl-2-methyl-2-isopropylpropane, 1,3-bis[bis(3,5-dimethyl-4-methoxyphenyl)phosphino]-1,3-diphenyl-2-ethyl-2-propylpropane, 1,3-bis[bis(3,5-dimethyl-4-methoxyphenyl)phosphino]-1,3-diphenyl-2-ethyl-2-isopropylpropane, and 1,3-bis[bis(3,5-dimethyl-4-methoxyphenyl)phosphino]-1,3-diphenyl-2-propyl-2-isopropylpropane.

Among them, SKEWPHOS, TolSKEWPHOS, 3,5-diEtSKEWPHOS, 4-t-BuSKEWPHOS, XylSKEWPHOS, and ModSKEWPHOS are preferable.

Diamine compound B in Formula (1) is represented by Formula (3).

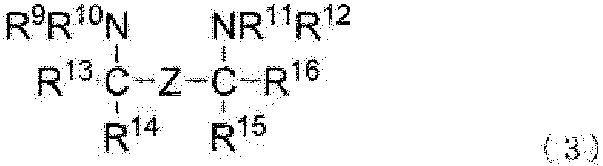

R⁹, R¹⁰, R¹¹, and R¹² are each independently, without particular limitation, for example, hydrogen, an aliphatic or alicyclic saturated or unsaturated hydrocarbon group, or a monocyclic or polycyclic aromatic or araliphatic hydrocarbon group, the hydrocarbon group optionally further having one or more substituents.

Examples of R⁹, R¹⁰, R¹¹, and R¹² above include hydrogen and a hydrocarbon group such as an alkyl group, an alkenyl group, an alkynyl group, a cycloalkyl group, a cycloalkenyl group, an aryl group, or an aralkyl group. From the viewpoint of catalytic activity, they are preferably hydrogen or an optionally substituted chain-form or cyclic hydrocarbon group having 1 to 30 carbons, and more preferably hydrogen, an alkyl group, a phenyl group, or a phenylalkyl group.

With regard to combination of R⁹, R¹⁰, R¹¹, and R¹², from the viewpoint of catalytic activity it is preferable that R⁹ is hydrogen and that R¹⁰, R¹¹, and R¹² are each independently hydrogen, an alkyl group, a phenyl group, or a phenylalkyl group, and it is particularly preferable that all of R⁹, R¹⁰, R¹¹, and R¹² are hydrogen.
R¹³, R¹⁴, R¹⁵, and R¹⁶ are each independently, without particular limitation, for example hydrogen, an aliphatic or alicyclic saturated or unsaturated hydrocarbon group, or a monocyclic or polycyclic aromatic or araliphatic hydrocarbon group, the hydrocarbon group optionally further having one or more substituents.

Examples of R¹³, R¹⁴, R¹⁵, and R¹⁶ above include hydrogen and a hydrocarbon group such as an alkyl group, an alkenyl group, an alkynyl group, a cycloalkyl group, a cycloalkenyl group, an aryl group, or an aralkyl group. From the viewpoint of catalytic activity and enantioselectivity, they are preferably hydrogen or an optionally substituted chain-form or cyclic hydrocarbon group having 1 to 30 carbons, more preferably hydrogen, a methyl group, an ethyl group, a propyl group, an isopropyl group, a phenyl group, or a substituted phenyl group, and particularly preferably hydrogen, a phenyl group or a substituted phenyl group.

With regard to combination of R¹³, R¹⁴, R¹⁵, and R¹⁶, from the viewpoint of catalytic activity and enantioselectivity it is preferable that R¹³ and R¹⁵ are hydrogen, R¹⁴ is hydrogen, a phenyl group, or a substituted phenyl group, and R¹⁶ is a phenyl group or a substituted phenyl group.

With regard to combination of R⁹, R¹⁰, R¹¹, R¹², R¹³, R¹⁴, R¹⁵, and R¹⁶, from the viewpoint of catalytic activity and enantioselectivity it is preferable that R⁹, R¹⁰, R¹¹, R¹², R¹³, and R¹⁵ are hydrogen, R¹⁴ is hydrogen, a phenyl group, or a substituted phenyl group, and R¹⁶ is a phenyl group or a substituted phenyl group.

The substituted phenyl group is preferably a phenyl group substituted with one to five groups selected from the group consisting of an alkyl group or alkoxy group having 1 to 5 carbons, a perfluoroalkyl group having 1 to 5 carbons, and a halogen.
The hydrocarbon group may optionally have one or more independently selected substituents such as an alkyl group, an alkenyl group, an alkynyl group, a cycloalkyl group, a cycloalkenyl group, an aryl group, a heteroaryl group, an alkoxy group, an ester group, an acyloxy group, a halogen atom, a nitro group, a cyano group, or a perfluoroalkyl group.

Examples of the alkyl group include a methyl group, an ethyl group, an *n*-propyl group, an isopropyl group, an *n*-butyl group, a *sec*-butyl group, an isobutyl group, and a *tert*-butyl group.
Examples of the alkenyl group include a vinyl group, a 1-propenyl group, an allyl group, an isopropenyl group, a 1-butenyl group, a crotyl group, a 3-butenyl group, an isobutenyl group, a methallyl group, and a 2-phenylvinyl group.
Examples of the alkynyl group include an ethynyl group, a 1-propynyl group, a propargyl group, a 1-butynyl group, a 2-butynyl group, a 3-butynyl group, a 3-methyl-1-butynyl group, and a 2-phenylethynyl group.

Examples of the cycloalkyl group include a cyclopropyl group, a cyclobutyl group, a cyclopentyl group, and a cyclohexyl group.
Examples of the cycloalkenyl group include a cyclopropenyl group, a cyclobutenyl group, a cyclopentenyl group, and a cyclohexenyl group.
Examples of the aryl group include a phenyl group, a tolyl group, a xylyl group, a ferrocenyl group, and a naphthyl group.

Examples of the aralkyl group include a benzyl group, a phenethyl group, and a phenylpropyl group.
Examples of the heteroaryl group include a pyridyl group, a pyrimidyl group, a pyrazyl group, a quinolyl group, an isoquinolyl group, a thienyl group, a furyl group, a pyrrolyl group, an indolyl group, an imidazolyl group, a pyrazolyl group, a thiazolyl group, and a triazolyl group.

Examples of the alkoxy group include a methoxy group, an ethoxy group, a propoxy group, and a butoxy group.
Examples of the ester group include a methoxycarbonyl group, an ethoxycarbonyl group, a butoxycarbonyl group, and a benzyloxycarbonyl group.
Examples of the acyloxy group include an acetoxy group, a trifluoroacetoxy group, an acryloxy group, and a methacryloxy group.
Examples of the halogen atom include a fluorine atom, a chlorine atom, a bromine atom, and an iodine atom.
Examples of the perfluoroalkyl group include a trifluoromethyl group and a pentafluoroethyl group.

Examples of Z include, but are not particularly limited to, an optionally substituted chain-form or cyclic hydrocarbon group having 1 to 10 carbons, and a single bond.
Examples of the optionally substituted hydrocarbon group having 1 to 10 carbons include a methylene group, an ethylene group, and a propylene group.

From the viewpoint of catalytic activity and enantioselectivity, it is preferably a single bond.
The optionally substituted hydrocarbon group having 1 to 10 carbons may optionally have one or more independently selected substituents such as an alkyl group, an alkenyl group, an alkynyl group, a cycloalkyl group, a cycloalkenyl group, an aryl group, a heteroaryl group, an alkoxy group, an ester group, an acyloxy group, a halogen atom, a nitro group, a cyano group, or a perfluoroalkyl group.

Examples of the alkyl group include a methyl group, an ethyl group, an *n*-propyl group, an isopropyl group, an *n*-butyl group, a *sec-*butyl group, an isobutyl group, and a *tert*-butyl group.
Examples of the alkenyl group include a vinyl group, a 1-propenyl group, an allyl group, an isopropenyl group, a 1-butenyl group, a crotyl group, a 3-butenyl group, an isobutenyl group, a methallyl group, and a 2-phenylvinyl group.
Examples of the alkynyl group include an ethynyl group, a 1-propynyl group, a propargyl group, a 1-butynyl group, a 2-butynyl group, a 3-butynyl group, a 3-methyl-1-butynyl group, and a 2-phenylethynyl group.

Examples of the cycloalkyl group include a cyclopropyl group, a cyclobutyl group, a cyclopentyl group, and a cyclohexyl group.
Examples of the cycloalkenyl group include a cyclopropenyl group, a cyclobutenyl group, a cyclopentenyl group, and a cyclohexenyl group.
Examples of the aryl group include a phenyl group, a tolyl group, a xylyl group, a ferrocenyl group, and a naphthyl group.

Examples of the heteroaryl group include a pyridyl group, a pyrimidyl group, a pyrazyl group, a quinolyl group, an isoquinolyl group, a thienyl group, a furyl group, a pyrrolyl group, an indolyl group, an imidazolyl group, a pyrazolyl group, a thiazolyl group, and a triazolyl group.

Examples of the alkoxy group include a methoxy group, an ethoxy group, a propoxy group, and a butoxy group.
Examples of the ester group include a methoxycarbonyl group, an ethoxycarbonyl group, a butoxycarbonyl group, and a benzyloxycarbonyl group.
Examples of the acyloxy group include an acetoxy group, a trifluoroacetoxy group, an acryloxy group, and a methacryloxy group.
Examples of the halogen atom include a fluorine atom, a chlorine atom, a bromine atom, and an iodine atom.
Examples of the perfluoroalkyl group include a trifluoromethyl group and a pentafluoroethyl group.

Specific examples of the diamine compound B represented by Formula (3) include DPEN: 1,2-diphenylethylenediamine, 2-F-DPEN: 1,2-di(2-fluorophenyl)ethylenediamine, 3-F-DPEN: 1,2-di(3-fluorophenyl)ethylenediamine, 4-F-DPEN: 1,2-di(4-fluorophenyl)ethylenediamine, 2-CF₃-DPEN: 1,2-di(2-trifluoromethylphenyl)ethylenediamine, 3-CF₃-DPEN: 1,2-di(3-trifluoromethylphenyl)ethylenediamine, 4-CF₃-DPEN: 1,2-di(4-trifluoromethylphenyl)ethylenediamine, 2-Me-DPEN: 1,2-di(2-methylphenyl)ethylenediamine, 3-Me-DPEN: 1,2-di(3-methylphenyl)ethylenediamine, 4-Me-DPEN: 1,2-di(4-methylphenyl)ethylenediamine, 2-MeO-DPEN: 1,2-di(2-methoxyphenyl)ethylenediamine, 3-MeO-DPEN: 1,2-di(3-methoxyphenyl)ethylenediamine, 4-MeO-DPEN: 1,2-di(4-methoxyphenyl)ethylenediamine, 3,5-Me₂-DPEN: 1,2-bis(3,5-dimethylphenyl)ethylenediamine, *N*-methyl-1,2-diphenylethylenediamine, *N*,*N*-dimethyl-1,2-diphenylethylenediamine, *N*,*N*'-dimethyl-1,2-diphenylethylenediamine, PEN: 1-phenylethylenediamine, EN: ethylenediamine, CYDN: 1,2-cyclohexanediamine, 1,2-cycloheptanediamine, 2,3-dimethylbutanediamine, and DAIPEN: 1-isopropyl-2,2-di(4-methoxyphenyl)ethylenediamine. Among them, DPEN, 4-F-DPEN, 4-CF₃-DPEN, 3-MeO-DPEN, 4-MeO-DPEN, 4-Me-DPEN, and PEN are preferable.

Furthermore, X, Y, and diphosphine compound A of a ruthenium complex represented by Formula (6) used in the present invention may be appropriately selected from the same kinds as those of Formula (1).

Examples of an organic ligand L contained in the ruthenium complex of Formula (6) include, but are not particularly limited to, an aromatic hydrocarbon such as toluene or xylene, an aliphatic hydrocarbon such as pentane or hexane, a halogen-containing hydrocarbon such as methylene chloride, an ether compound such as diethyl ether or tetrahydrofuran, an alcohol compound such as methanol, ethanol, 2-propanol, butanol, or benzyl alcohol, a ketone compound such as acetone, methyl ethyl ketone, or cyclohexyl ketone, and a heteroelement-containing compound such as acetonitrile, *N,N*-dimethylformamide, *N-*methylpyrrolidone, dimethyl sulfoxide, or triethylamine.

In Formula (6) n is any number between 0 and 2.
Methods for synthesizing ruthenium complexes represented by Formulae (1) and (6) are described in Japanese Patent No. 3566955, JP, A, 2004-238306, and the like. Specifically, a ruthenium complex represented by Formula (6) may be synthesized by a reaction between a diphosphine compound and a ruthenium complex as a starting material. Further reacting a ruthenium complex represented by Formula (6) with a diamine compound enables a ruthenium complex represented by Formula (1) to be synthesized.

An imine compound as a starting material in the present invention is represented by Formula (4).

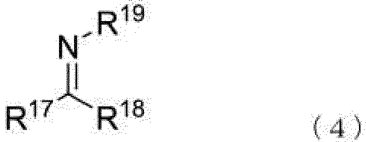

R¹⁷, R¹⁸, and R¹⁹ are not particularly limited and are for example hydrogen or an organic group such as an alkyl group having 1 to 20 carbons, an alkenyl group having 2 to 20 carbons, an alkynyl group having 2 to 20 carbons, a cycloalkyl group having 3 to 20 carbons, a cycloalkenyl group having 3 to 20 carbons, an aryl group, a heteroaryl group, an aralkyl group, or a heteroaralkyl group. R¹⁸ may be different from R¹⁷.

Examples of R¹⁷, R¹⁸, and R¹⁹ include organic groups such as an aryl group such as a phenyl group or a naphthyl group, a heteroaryl group such as pyridine, pyrimidine, pyrazine, quinoline, isoquinoline, thiophene, benzothiophene, furan, benzofuran, pyrrole, indole, imidazole, pyrazole, thiazole, or triazole, an aralkyl group in which an alkyl group having 1 to 4 carbons is substituted with an aryl group, and a heteroaralkyl group in which an alkyl group having 1 to 4 carbons is substituted with a heteroaryl group. From the viewpoint of reactivity and enantioselectivity, it is preferable that R¹⁷ is an alkenyl group, an alkynyl group, a cycloalkenyl group, an aryl group, or a heteroaryl group, R¹⁸ is an alkyl group, a cycloalkyl group, an alkenyl group, a cycloalkenyl group, or an aryl group, and R¹⁹ is an alkyl group, an alkenyl group, an alkynyl group, an aryl group, a heteroaryl group, or an aralkyl group, and it is more preferable that R¹⁷ is an alkenyl group, an aryl group, or a heteroaryl group, R¹⁸ is an alkyl group or a cycloalkyl group, and R¹⁹ is an alkyl group, an aryl group, or an aralkyl group.

Furthermore, two of R¹⁷, R¹⁸, and R¹⁹ may together form a ring, and examples of the ring include, but are not particularly limited to, a cyclopentane ring and a cyclohexane ring. From the viewpoint of reactivity and enantioselectivity one in which R¹⁷ and R¹⁸ together form a ring is preferable.

The organic group and the optionally substituted ring formed from two of R¹⁷, R¹⁸, and R¹⁹ above each independently and optionally have various types of acceptable substituents such as an alkyl group, an alkenyl group, an alkynyl group, a cycloalkyl group, a cycloalkenyl group, an aryl group, a heteroaryl group, an alkoxy group, an ester group, an acyloxy group, a halogen atom, a nitro group, a cyano group, a perfluoroalkyl group, or an amino group.

Examples of the alkyl group include a methyl group, an ethyl group, an *n*-propyl group, an isopropyl group, an *n*-butyl group, a *sec*-butyl group, an isobutyl group, and a *tert*-butyl group.
Examples of the alkenyl group include a vinyl group, a 1-propenyl group, an allyl group, an isopropenyl group, a 1-butenyl group, a crotyl group, a 3-butenyl group, an isobutenyl group, a methallyl group, and a 2-phenylvinyl group.
Examples of the alkynyl group include an ethynyl group, a 1-propynyl group, a propargyl group, a 1-butynyl group, a 2-butynyl group, a 3-butynyl group, a 3-methyl-1-butynyl group, and a 2-phenylethynyl group.

Examples of the cycloalkyl group include a cyclopropyl group, a cyclobutyl group, a cyclopentyl group, and a cyclohexyl group.
Examples of the cycloalkenyl group include a cyclopropenyl group, a cyclobutenyl group, a cyclopentenyl group, and a cyclohexenyl group.
Examples of the aryl group include a phenyl group, a tolyl group, a xylyl group, a ferrocenyl group, and a naphthyl group.

Examples of the heteroaryl group include a pyridyl group, a pyrimidyl group, a pyrazyl group, a quinolyl group, an isoquinolyl group, a thienyl group, a furyl group, a pyrrolyl group, an indolyl group, an imidazolyl group, a pyrazolyl group, a thiazolyl group, and a triazolyl group.

Examples of the alkoxy group include a methoxy group, an ethoxy group, a propoxy group, and a butoxy group.
Examples of the ester group include a methoxycarbonyl group, an ethoxycarbonyl group, a butoxycarbonyl group, and a benzyloxycarbonyl group.
Examples of the acyloxy group include an acetoxy group, a trifluoroacetoxy group, an acryloxy group, and a methacryloxy group.
Examples of the halogen atom include a fluorine atom, a chlorine atom, a bromine atom, and an iodine atom.
Examples of the perfluoroalkyl group include a trifluoromethyl group and a pentafluoroethyl group.

The imine compound may be synthesized by dehydration-condensation of a carbonyl compound and an amine compound.

An optically active amine compound as a product in the present invention is represented by Formula (5). 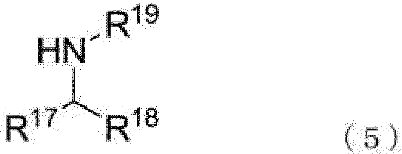 R¹⁷, R¹⁸, and R¹⁹ in the optically active amine compound correspond to R¹⁷, R¹⁸, and R¹⁹ in the imine compound represented by Formula (4), which is a starting material.

In certain embodiments, a method of the present invention is carried out by mixing a ruthenium complex represented by Formula (1), an imine compound represented by Formula (4), and a base in an aprotic solvent or without a solvent under pressurized hydrogen to thus hydrogenate the imine compound. In other embodiments, a method of the present invention is carried out by mixing a ruthenium complex represented by Formula (6), a diamine compound represented by Formula (3), an imine compound represented by Formula (4), and a base in an aprotic solvent or without a solvent under pressurized hydrogen to thus hydrogenate the imine compound.

In the hydrogenation, the amount of ruthenium complex used as a catalyst is not particularly limited, but taking practicality into consideration the molar ratio of the imine compound relative to the ruthenium complex when expressed as S/C (S is substrate and C is catalyst) may be in the range of 10 to 100,000, and is preferably in the range of 50 to 50,000.

The base used in the hydrogenation reaction is not particularly limited, but examples thereof include an alkali metal or alkaline earth metal salt, a quaternary ammonium salt, and a tertiary amine. From the viewpoint of reactivity, cost, simplicity, and the like, it is preferably one or more selected from the group consisting of KOH, KOCH₃, KOC₂H₅, KOCH(CH₃)₂, KOC(CH₃)₃, KC₁₀H₈, NaOH, NaOCH₃, NaOC₂H₅, NaOCH(CH₃)₂, NaOC(CH₃)₃, LiOH, LiOCH₃, LiOC₂H₅, LiOCH(CH₃)₂, LiOC(CH₃)₃, N(CH₃)₄OH, N(C₂H₅)₄OH, N(CH₃)₄OCH₃, N(CH₃)₄OC₂H₅, N(C₂H₅)₃, and DBU (diazabicycloundecene).

The amount of base used may be in the range of 0.001 to 10 molar with respect to the volume of the entire reaction system, and is preferably in the range of 0.01 to 1 molar.

Examples of the aprotic solvent include, but are not particularly limited to, an aromatic hydrocarbon solvent such as toluene, benzene, or xylene, an ether-based solvent such as tetrahydrofuran, *tert*-butyl methyl ether, or diethyl ether, an aliphatic hydrocarbon solvent such as pentane, hexane, or heptane, an ester-based solvent such as ethyl acetate or isopropyl acetate, a halogen-containing hydrocarbon solvent such as methylene chloride or chloroform, and a heteroelement-containing solvent such as *N,N*-dimethylformamide, dimethyl sulfoxide, acetonitrile, or *N*-methylpyrrolidone, these being used singly or in a combination of two or more. It is also possible to use a mixed solvent of an aprotic solvent exemplified above and another solvent.

As described above, it has been confirmed that in the production methods of the present invention, the hydrogenation reaction hardly proceeds at all in the presence of a protic solvent such as an alcohol. Because of this, in the production methods of the present invention, it is essential for the reaction to be carried out in the presence of an aprotic solvent or under solvent-free conditions.

From the viewpoint of the efficiency of progress of the reaction, the solvent is preferably one or more selected from the group consisting of an aromatic hydrocarbon solvent, an ether-based solvent, an aliphatic hydrocarbon solvent, and an ester-based solvent, and is particularly preferably one or more selected from the group consisting of toluene, benzene, tetrahydrofuran, and *tert*-butyl methyl ether.

The amount of the aprotic solvent is not particularly limited, but from the viewpoint of solubility of the imine compound and cost, it may be no greater than 90 wt% relative to the weight of the entire reaction system, and when the imine compound is a liquid a reaction may be carried out without a solvent.

The hydrogen pressure in the hydrogenation under pressure is not particularly limited, but it may be carried out in the range of 1 to 200 atm, and when cost is taken into consideration it is preferably in the range of 5 to 150 atm. When the pressure of hydrogen is too low, there is the drawback that the reaction speed decreases.

The reaction temperature is not particularly limited, but when cost is taken into consideration it may be carried out at - 50°C to 100°C, and preferably in the range of -30°C to 80°C. When the reaction temperature is too low, the reaction speed decreases, and when it is too high there is the drawback that the catalyst is deactivated and the reaction stops before completion.

Since the reaction time depends on the type of imine compound, the concentration, S/C, reaction conditions such as temperature and hydrogen pressure, and the type of ruthenium complex, conditions may be set so that the reaction is complete in a few minutes to a few days, and it is particularly preferable for conditions to be set so that the reaction is complete in 1 to 24 hours.
The hydrogenation reaction of an imine compound in the present invention may be carried out by any reaction mode such as a batch method or a continuous method.

### [Examples]

The present invention is explained below in further detail by reference to Examples. Needless to say, the present invention is not limited by the Examples below.
In the Examples below, all of the reactions were carried out under an atmosphere of an inert gas such as argon gas or nitrogen gas. NMR was measured using an ECX-400P (400 MHz, JEOL). ¹H NMR and ¹³C NMR used tetramethylsilane as an internal reference substance, and its signal was defined as δ = 0 (δ is chemical shift). Optical purity was measured using high performance liquid chromatography (HPLC) with CHIRALCEL OD-H (0.46 cm x 25 cm), CHIRALCEL OJ-H (0.46 cm x 25 cm), and CHIRALPAK IA (0.46 cm x 25 cm) (Daicel). Specific rotation was measured using a P-2200 (JASCO Corporation).

The structures of imine compounds used in Examples 1 to 23, 41, and 42 and Comparative Examples 1 to 4 are as shown below.

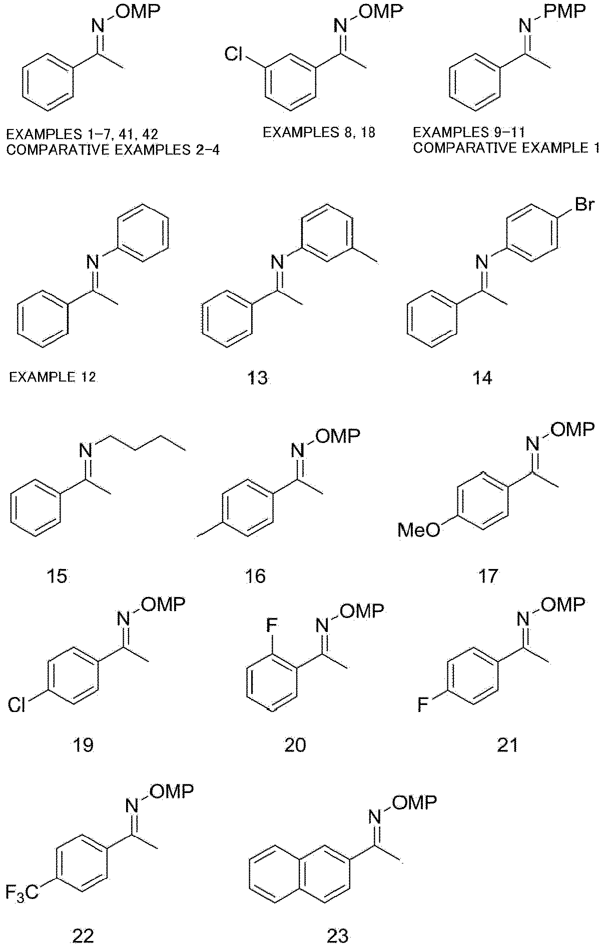

The structures of imine compounds used in Examples 24 to 39 and 43 are as shown below.

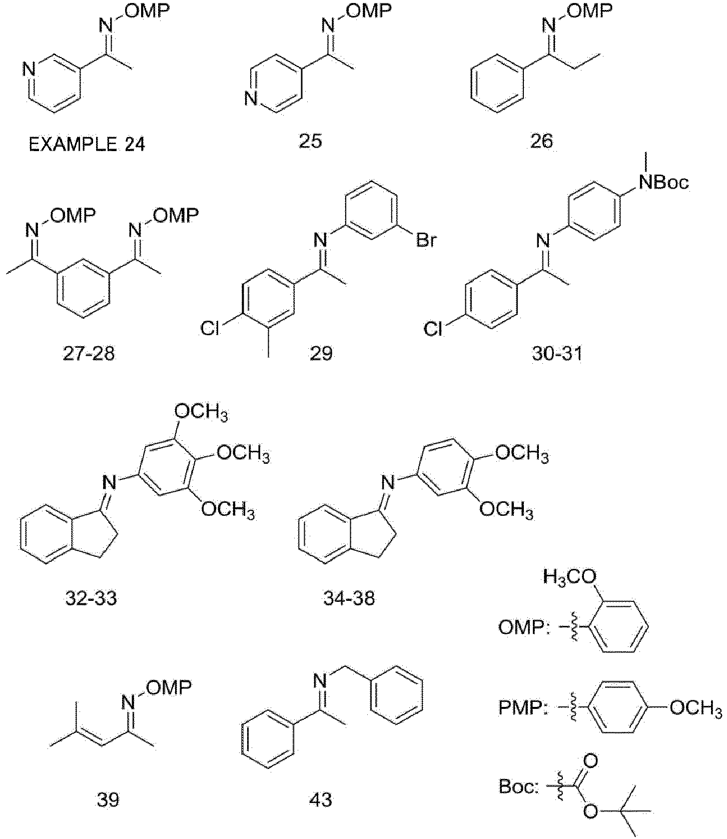

The structures of ruthenium complexes used in Examples 1 to 43 and Comparative Example 4 are as shown below. 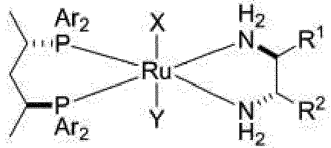 RuBr₂[(*S*,*S*)-xylskewphos][(*S*,*S*)-dpen]:
X = Y = Br, Ar = 3,5-(CH₃)₂C₆H₃, R¹ = R² = Ph.
RuBr₂[(*S*,*S*)-skewphos][(*S*,*S*)-dpen]:
X=Y=Br,Ar= R¹= R²=Ph.
RuBr₂[(*S*,*S*)-tolskewphos][(*S*,*S*)-dpen]:
X = Y = Br, Ar = 4-CH₃C₆H₄, R¹ = R² = Ph.
RuCl₂[(*S*,*S*)-xylskewphos][(*S*,*S*)-dpen]:
X = Y = Cl, Ar = 3,5-(CH₃)₂C₆H₃, R¹ = R² = Ph.
Rul₂[(*S*,*S*)-xylskewphos][(*S*,*S*)-dpen]:
X = Y = I, Ar = 3,5-(CH₃)₂C₆H3, R¹ = R² = Ph.
RuBr₂[(*S*,*S*)-xylskewphos][(*S*,*S*)-4-CF₃dpen]:
X = Y = Br, Ar = 3,5-(CH₃)₂C₆H₃, R¹ = R² = 4-CF₃,C₆H₄,
RuBr₂[(*S*,*S*)-modskewphos][(*S*,*S*)-4-CF₃dpen]:
X = Y = Br, Ar = 3,5-(CH₃)₂-4-(OCH₃)C₆H₂, R¹ = R² = 4-CF₃C₆H₄.
RuBr₂[(*S*,*S*)-xylskewphos][(*S*,*S*)-4-Fdpen]:
X = Y = Br, Ar= 3,5-(CH₃)₂C₆H₃, R¹ = R² = 4-FC₆H₄.
RuBr₂[(*S*,*S*)-xylskewphos][(*S*,*S*)-4-Medpen]:
X = Y = Br, Ar = 3,5-(CH₃)₂C₆H₃, R¹ = R² = 4-CH₃C₆H₄.
RuBr₂[(*S*,*S*)-xylskewphos][(*S*,*S*)-3Medpen]:
X = Y = Br, Ar = 3,5.(CH₃)₂C₆H₃, R¹ = R² = 3-CH₃C₆H₄.
RUH(BH₄)[(S,S)-xylskewphos][(*S,S*)-dpen]:
X = H, Y = BH₄, Ar = 3,5-(CH₃)₂C₆H₃, R¹ = R² = Ph.
RuBr₂[(*S*,*S*)-xylskewphos][(*S*)-dpen]:
X = Y = Br, Ar = 3,5-(CH₃)₂C₆H₃, R¹ = H, R² = Ph.

The structures of ruthenium complexes used in Comparative Examples 1 to 3 are as shown below.

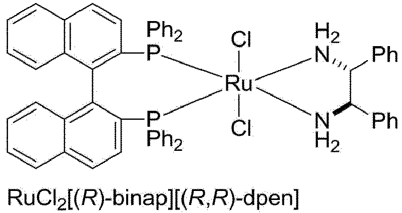

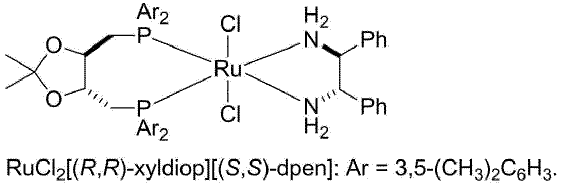

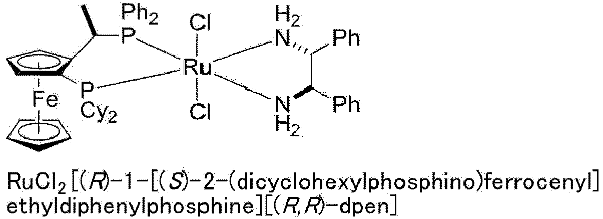

### [Example 1]

### Synthesis of (R)-N-(2-methoxyphenyl)-1-phenylethylamine by asymmetric hydrogenation of N-(1-phenylethylidene)-2-methoxyaniline

A glass autoclave was charged with RuBr₂[(*S*,*S*)-xylskewphos][(*S,S*)-dpen] (0.51 mg, 0.5 µmol), N-(1-phenylethylidene)-2-methoxyaniline (563 mg, 2.5 mmol), and KOC(CH₃)₃ (9.6 mg, 86 µmol) and flushed with argon. 0.63 mL of toluene was added thereto, and the autoclave was degassed and then flushed with hydrogen. It was charged with hydrogen until the pressure reached 10 atm, and a reaction was started by stirring in a water bath at 40°C. 24 hours later the pressure was returned to normal pressure. From ¹H NMR and HPLC of the product, it was found that 99% ee (*R*)-*N*-(2-methoxyphenyl)-1-phenylethylamine was formed in a yield of >99%. The spectral data of the amine compound obtained were as follows. ¹H NMR (400 MHz, CDCl₃) δ 1.55 (d, J = 6.4 Hz, 3H, CH₃), 3.88 (s, 3H, OCH₃), 4.47 (q, 6.4 Hz, 1H, CHNH), 4.66 (br, 1H, NH), 6.34-6.77 (m, 4H, aromatic H), 7.19-7.38 (m, 5H, aromatic H); ¹³C NMR (100 MHz, CDCl₃) δ 25.1, 53.4, 55.4, 109.3, 111.2, 116.4, 121.1, 125.9, 126.8, 128.6, 145.4, 146.6; HPLC (column, CHIRALCEL OD-H; solvent, hexane/2-propanol = 99/1; flow rate, 0.5 mL/min; temperature, 30°C; UV wavelength, 254 nm; t_{R} of (S)-N-(2-methoxyphenyl)-1-phenylethylamine 15.4 min.; t_{R} of (*R*)-*N*-(2-methoxyphenyl)-1-phenylethylamine 19.9 min.); specific rotation [α]²⁵_{D} -38.7° (c 1.01, CHCl₃); literature value, [α]²⁵_{D} -32.3° (c 1.03, CHCl₃, 97% ee (R)) J. Am. Chem. Soc. 2009, 131, 8358.

### [Example 2]

### Synthesis of (R)-N-(2-methoxyphenyl)-1-phenylethylamine by asymmetric hydrogenation of N-(1-phenylethylidene)-2-methoxyaniline

A glass autoclave was charged with RuBr₂[(*S*,*S*)-xylskewphos] (0.41 mg, 0.5 µmol), (*S*,*S*)-DPEN (0.11 mg, 0.5 µmol), *N*-(1-phenylethylidene)-2-methoxyaniline (451 mg, 2.0 mmol), and KOC(CH₃)₃ (7.7 mg, 68 µmol) and flushed with argon. 0.5 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 99% ee (*R*)-*N*-(2-methoxyphenyl)-1-phenylethylamine was formed in a yield of >99%.

### [Examples 3 and 4]

Reactions were carried out under the same conditions as for Example 1 except that the ruthenium complex, S/C, and the reaction time were changed, thus synthesizing (R)-N-(2-methoxyphenyl)-1-phenylethylamine. The results are summarized in Table 1.

**[Table 1]**

| | | | |
|---|---|---|---|
| 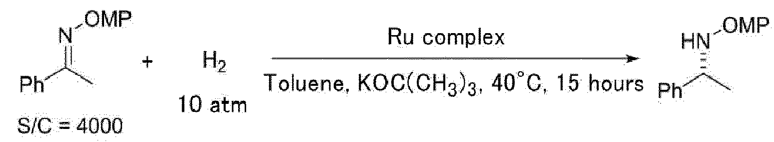 | | | |
| Substrate concentration 2.20 M | | | |
| Base concentration 75 mM | | | |

| Example | Ru complex | Yield (%) | ee (%) |
|---|---|---|---|
| 3 | RuBr₂[(*S*,*S*)-skewphos][(*S*,*S*)-dpen] | >99 | 99 |
| 4 | RuBr₂[(*S*,*S*)-tolskewphos][(*S*,*S*)-dpen] | 60 | 98 |

| | | | |
|---|---|---|---|
| OMP: *o*-methoxyphenyl group | | | |

### [Examples 5 to 7]

Reactions were carried out under the same conditions as for Example 1 except that the solvent, S/C, the substrate concentration, the base concentration, and the reaction time were changed, thus synthesizing (*R*)-*N*-(2-methoxyphenyl)-1-phenylethylamine. The results are summarized in Table 2.

**[Table 2]**

| | | | |
|---|---|---|---|
| 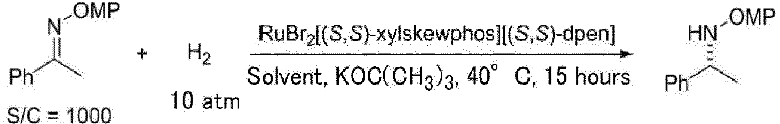 | | | |
| Substrate concentration 0.83 M | | | |
| Base concentration 42 mM | | | |

| Example | Solvent | Yield (%) | ee (%) |
|---|---|---|---|
| 5 | Benzene | >99 | 99 |
| 6 | *tert*-Butyl methyl ether | >99 | 99 |
| 7 | Tetrahydrofuran | 88 | 98 |

### [Example 8]

### Synthesis of (-)-N-(2-methoxyphenyl)-1-(3-chlorophenyl)ethylamine by asymmetric hydrogenation of N-[1-(3-chlorophenyl)ethylidene]-2-methoxyaniline

A glass autoclave was charged with RuBr₂[(*S*,*S*)-xylskewphos] [(*S*,*S*)-dpen] (1.03 mg, 1 µmol) and KOC(CH₃)₃ (17 mg, 150 µmol) and flushed with argon. *N*-[1-(3-Chlorophenyl)ethylidene]-2-methoxyaniline (0.68 mL, 3 mmol) was added thereto, and the autoclave was flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 98% ee (-)-N-(2-methoxyphenyl)-1-(3-chlorophenyl)ethylamine was formed in a yield of 37%. The reaction proceeded with high enantioselectivity under solvent-free conditions. The spectral data of the amine compound obtained were as follows. ¹H NMR (400 MHz, CDCl₃) δ 1.53 (d, J = 6.9 Hz, 3H, CH₃), 3.89 (s, 3H, OCH₃), 4.42 (q, 6.9 Hz, 1H, CHNH), 4.60 (br, 1H, NH), 6.28 (dd, J = 1.8 Hz, 7.8 Hz, 1H, aromatic H), 6.60-6.78 (m, 3H, aromatic H), 7.17-7.36 (m, 4H, aromatic H); ¹³C NMR (100 MHz, CDCl₃) δ 25.2, 53.1, 55.4, 109.3, 111.0, 116.6, 121.1, 124.0, 126.0, 127.0, 129.9, 134.5, 136.9, 146.5, 147.9; HPLC (column, CHIRALCEL OD-H; solvent, hexane/2-propanol = 99/1; flow rate, 1.0 mL/min; temperature, 30°C; UV wavelength, 254 nm; t_{R} of (+)-*N*-(2-methoxyphenyl)-1-(3-chlorophenyl)ethylamine 7.5 min.; t_{R} of (-)-N-(2-methoxyphenyl)-1-(3-chlorophenyl)ethylamine 10.7 min.); specific rotation [α]²⁵_{D} -43.0° (c 1.03, CHCl₃).

### [Example 9]

### Synthesis of (R)-N-(4-methoxyphenyl)-1-phenylethylamine by asymmetric hydrogenation of N-(1-phenylethylidene)-4-methoxyaniline

A glass autoclave was charged with RuBr₂[(*S*,*S*)-xylskewphos][(*S,S*)-dpen] (1.03 mg, 1 µmol), *N*-(1-phenylethylidene)-4-methoxyaniline (676 mg, 3 mmol), and KOC(CH₃)₃ (17 mg, 150 µmol) and flushed with argon. 3 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 95% ee (*R*)-N-(4-methoxyphenyl)-1-phenylethylamine was formed in a yield of >99%. The spectral data of the amine compound obtained were as follows. ¹H NMR (400 MHz, CDCl₃) δ 1.50 (d, J = 6.9 Hz, 3H, CH₃), 3.69 (s, 3H, OCH₃), 3.78 (br, 1H, NH), 4.41 (q, 6.9 Hz, 1H, CHNH), 6.45-6.71 (m, 4H, aromatic H), 7.19-7.37 (m, 5H, aromatic H); ¹³C NMR (100 MHz, CDCl₃) δ 25.1, 54.3, 55.7, 114.6, 114.8, 125.9, 126.8, 128.6, 141.5, 145.4, 152.0; HPLC (column, CHIRALCEL OD-H; solvent, hexane/2-propanol = 99/1; flow rate, 1.0 mL/min; temperature, 30°C; UV wavelength, 254 nm; t_{R} of (*R*)-*N*-(4-methoxyphenyl)-1-phenylethylamine 11.7 min.; t_{R} of (*S*)-*N*-(4-methoxyphenyl)-1-phenylethylamine 13.0 min.); specific rotation [α]²⁵_{D} +4.6° (c 1.01, CHCl₃); literature value, [α]²⁵_{D} +1.4° (c 1.00, CHCl₃, 71% ee (R)) J. Am. Chem. Soc. 2009, 131, 8358.

### [Examples 10 and 11]

### Synthesis of (R)-N-(4-methoxyphenyl)-1-phenylethylamine by asymmetric hydrogenation of N-(1-phenylethylidene)-4-methoxyaniline

Reactions were carried out under the same conditions as for Example 9 except that the Ru complex and S/C were changed, thus synthesizing (*R*)-*N*-(4-methoxyphenyl)-1-phenylethylamine. The results are summarized in Table 3.

**[Table 3]**

| | | | |
|---|---|---|---|
| 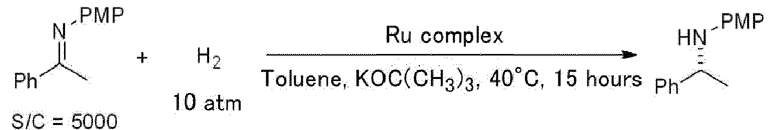 | | | |
| Substrate concentration 0.83 M | | | |
| Base concentration 42 mM | | | |

| Example | Ru complex | Yield (%) | ee (%) |
|---|---|---|---|
| 10 | RuCl₂[(*S,S*)-xylskewphos][(*S*,*S*)-dpen] | 47 | 95 |
| 11 | Rul₂[(*S,S*)-xylskewphos][(*S,S*)-dpen] | 26 | 94 |

| | | | |
|---|---|---|---|
| PMP: *p*-methoxyphenyl group | | | |

### [Example 12]

### Synthesis of (R)-N-phenyl-1-phenylethylamine by asymmetric hydrogenation of N-(1-phenylethylidene)aniline

A glass autoclave was charged with RuBr₂[(*S,S*)-xylskewphos][(*S,S*)-dpen] (1.03 mg, 1 µmol), *N*-(1-phenylethylidene)aniline (391 mg, 2 mmol), and KOC(CH₃)₃ (7.7 mg, 68 µmol) and flushed with argon. 0.55 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 96% ee (*R*)-*N*-phenyl-1-phenylethylamine was formed in a yield of >99%. The spectral data of the amine compound obtained were as follows. ¹H NMR (400 MHz, CDCl₃) δ 1.51 (d, J = 6.9 Hz, 3H, CH₃), 4.01 (br, 1H, NH), 4.48 (q, 6.9 Hz, 1H, CHNH), 6.50-6.66 (m, 3H, aromatic H), 7.07-7.38 (m, 7H, aromatic H); ¹³C NMR (100 MHz, CDCl₃) δ 25.0, 53.4, 113.3, 117.2, 125.8, 126.8, 128.6, 129.1, 145.2, 147.3; HPLC (column, CHIRALCEL OD-H; solvent, hexane/2-propanol = 99/1; flow rate, 1.0 mL/min; temperature, 30°C; UV wavelength, 254 nm; t_{R} of (*S*)-*N*-phenyl-1-phenylethylamine, 11.3 min.; t_{R} of (*R*)-*N*-phenyl-1-phenylethylamine 12.9 min.); specific rotation [α]²⁵_{D} -3.8° (c 1.01, CHCl₃); literature value, [α]²⁵_{D} - 4.5° (c 1.05, CHCl₃, 87% ee (R)) J. Am. Chem. Soc. 2009, 131, 8358.

### [Example 13]

### Synthesis of (+)-N-(3-methylphenyl)-1-phenylethylamine by asymmetric hydrogenation of N-(1-phenylethylidene)-3-methylaniline

A glass autoclave was charged with RuBr₂[(*S,S*)-xylskewphos][(*S,S*)-dpen] (0.51 mg, 0.5 µmol) and KOC(CH₃)₃ (5.7 mg, 51 µmol) and flushed with argon. 0.37 mL of toluene and N-(1-phenylethylidene)-3-methylaniline (0.31 mL, 1.5 mmol) were added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 98% ee (+)-*N*-(3-methylphenyl)-l-phenylethylamine was formed in a yield of 99%. The spectral data of the amine compound obtained were as follows. ¹H NMR (400 MHz, CDCl₃) δ 1.50 (d, J = 6.9 Hz, 3H, CHCH₃) , 2.21 (s, 3H, CH₃), 3.96 (br, 1H, NH), 4.47 (q, 6.9 Hz, 1H, CHNH), 6.29-6.48 (m, 3H, aromatic H), 6.97 (t, 7.8 Hz, 1H, aromatic H), 7.20-7.38 (m, 5H, aromatic H); ¹³C NMR (100 MHz, CDCl₃) δ 21.6, 24.9, 53.4, 110.3, 114.1, 118.2, 125.8, 126.8, 128.6, 129.0, 138.8, 145.3, 147.3; HPLC (column, CHIRALCEL OD-H; solvent, hexane/2-propanol = 99.1/0.9; flow rate, 1.0 mL/min; temperature, 30°C; UV wavelength, 254 nm; t_{R} of (-)-N-(3-methylphenyl)-1-phenylethylamine 9.5 min.; t_{R} of (+)-N-(3-methylphenyl)-1-phenylethylamine 12.2 min.); specific rotation [α]²⁵_{D} +5.50 (c 1.01, CHCl₃).

### [Example 14]

### Synthesis of (R)-N-(4-bromophenyl)-1-phenylethylamine by asymmetric hydrogenation of N-(1-phenylethylidene)-4-bromoaniline

A glass autoclave was charged with RuBr₂[(*S,S*)-xylskewphos][(*S,S*)-dpen] (1.03 mg, 1 µmol), *N*-(1-phenylethylidene)-4-bromoaniline (411 mg, 1.5 mmol), and KOC(CH₃)₃ (5.6 mg, 50 µmol) and flushed with argon. 0.3 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 93% ee (*R*)-*N*-(4-bromophenyl)-1-phenylethylamine was formed in a yield of >99%. The spectral data of the amine compound obtained were as follows. ¹H NMR (400 MHz, CDCl₃) δ 1.51 (d, J = 6.4 Hz, 3H, CH₃), 4.06 (br, 1H, NH), 4.43 (q, 6.4 Hz, 1H, CHNH), 6.35-6.39 (m, 2H, aromatic H), 7.13-7.34 (m, 7H, aromatic H); ¹³C NMR (100 MHz, CDCl₃) δ 24.9, 53.5, 108.9, 114.9, 125.7, 127.0, 128.7, 131.8, 144.6, 146.2; HPLC (column, CHIRALCEL OD-H; solvent, hexane/2-propanol = 99/1; flow rate, 0.5 mL/min; temperature, 30°C; UV wavelength, 254 nm; t_{R} of (*R*)-*N*-(4-bromophenyl)-1-phenylethylamine 14.9 min.; t_{R} of *(S)-N-*(4-bromophenyl)-1-phenylethylamine 19.1 min.); specific rotation [α]²⁵_{D} +16.0° (c 1.02, CHCl₃); literature value, [α]¹⁵_{D} +7.6° (c 0.53, CHCl₃, 97% ee (*R*)) Org. Lett. 2009, 11, 4180.

### [Example 15]

### Synthesis of (+)-N-butyl-1-phenylethylamine by asymmetric hydrogenation of N-(1-phenylethylidene)butylamine

A glass autoclave was charged with RuBr₂[(*S,S*)-xylskewphos][(*S,S*)-dpen] (2.05 mg, 2 µmol) and KOC(CH₃)₃ (7.7 mg, 68 µmol) and flushed with argon. 0.53 mL of toluene and *N*-(1-phenylethylidene)butylamine (0.38 mL, 2 mmol) were added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR of the product, it was found that (+)-N-butyl-1-phenylethylamine was formed in a yield of 96%. The spectral data of the amine compound obtained were as follows. ¹H NMR (400 MHz, CDCl₃) δ 0.87 (t, J = 7.3 Hz, 3H, (CH₂)₂CH3), 1.26-1.33 (m, 2H, CH₂), 1.35 (d, J = 6.4 Hz, 3H, CHCH₃), 1.40-1.49 (m, 2H, CH₂), 2.38-2.53 (m, 2H, CH₂), 3.75 (q, J = 6.4 Hz, 1H, CHNH), 7.21-7.34 (m, 5H, aromatic H); ¹³C NMR (100 MHz, CDCl₃) δ 14.0, 20.5, 24.3, 32.4, 47.6, 58.4, 126.6, 126.8, 128.4, 145.9; specific rotation [α]²⁵_{D} +48.6° (c 1.02, CHCl₃).
The (+)-N-Butyl-1-phenylethylamine thus obtained was reacted with acetic anhydride to thus give the derivative N-acetyl-N-butyl-1-phenylethylamine, and it was found from HPLC that the optical purity was 97% ee. HPLC (column, CHIRALPAK IA; solvent, hexane/2-propanol = 95/5; flow rate, 1.0 mL/min; temperature, 30°C; UV wavelength, 210 nm; t_{R} of N-acetyl-N-butyl-1-phenylethylamine 8.2 min., 9.1 min.).

### [Example 16]

### Synthesis of (-)-N-(2-methoxyphenyl)-1-(4-methylphenyl)ethylamine by asymmetric hydrogenation of N-[1-(4-methylphenyl)ethylidene]-2-methoxyaniline

A glass autoclave was charged with RuBr₂[(*S,S*)-xylskewphos][(*S,S*)-dpen] (0.51 mg, 0.5 µmol) and KOC(CH₃)₃ (5.7 mg, 51 µmol) and flushed with argon. 0.34 mL of toluene and N-[1-(4-methylphenyl)ethylidene]-2-methoxyaniline (0.34 mL, 1.5 mmol) were added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 98% ee (-)-*N*-(2-methoxyphenyl)-1-(4-methylphenyl)ethylamine was formed in a yield of >99%. The spectral data of the amine compound obtained were as follows. ¹H NMR (400 MHz, CDCl₃) δ 1.52 (d, J = 6.9 Hz, 3H, CHCH₃), 2.31 (s, 3H, CH₃), 3.87 (s, 3H, OCH₃), 4.44 (q, 6.9 Hz, 1H, CHNH), 4.60 (br, 1H, NH), 6.35 (dd, J = 1.4 Hz, 7.8 Hz, 1H, aromatic H), 6.57-6.76 (m, 3H, aromatic H), 7.10-7.26 (m, 4H, aromatic H); ¹³C NMR (100 MHz, CDCl₃) δ 21.0, 25.2, 53.0, 55.4, 109.2, 111.0, 116.2, 121.1, 125.7, 129.2, 136.2, 137.3, 142.4, 146.5; HPLC (column, CHIRALCEL OJ-H; solvent, hexane/2-propanol = 99/1; flow rate, 1.0 mL/min; temperature, 30°C; UV wavelength, 254 nm; t_{R} of (-)-*N*-(2-methoxyphenyl)-1-(4-methylphenyl)ethylamine 17.3 min.; t_{R} of (+)-N-(2-methoxyphenyl)-1-(4-methylphenyl)ethylamine 27.4 min.); specific rotation [α]²⁵_{D} -24.0° (c 1.02, CHCl₃).

### [Example 17]

### Synthesis of (-)-N-(2-methoxyphenyl)-1-(4-methoxyphenyl)ethylamine by asymmetric hydrogenation of N-[1-(4-methoxyphenyl)ethylidene]-2-methoxyaniline

A glass autoclave was charged with RuBr₂[(*S,S*)-xylskewphos][(*S,S*)-dpen] (0.51 mg, 0.5 µmol), *N*-[1-(4-methoxyphenyl)ethylidene]-2-methoxyaniline (383 mg, 1.5 mmol), and KOC(CH₃)₃ (5.7 mg, 51 µmol) and flushed with argon. 0.33 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 98% ee (-)-*N*-(2-methoxyphenyl)-1-(4-methoxyphenyl)ethylamine was formed in a yield of >99%. The spectral data of the amine compound obtained were as follows. ¹H NMR (400 MHz, CDCl₃) δ 1.52 (d, J = 6.9 Hz, 3H, CH₃), 3.77 (s, 3H, OCH₃), 3.87 (s, 3H, OCH₃), 4.42 (q, 6.9 Hz, 1H, CHNH), 4.58 (br, 1H, NH), 6.35-6.86 (m, 6H, aromatic H), 7.26-7.29 (m, 2H, aromatic H); ¹³C NMR (100 MHz, CDCl₃) δ 25.1, 52.6, 55.2, 55.4, 109.2, 111.0, 113.9, 116.2, 121.1, 126.8, 137.2, 137.5, 146.5, 158.4; HPLC (column, CHIRALCEL OD-H; solvent, hexane/2-propanol = 99/1; flow rate, 1.0 mL/min; temperature, 30°C; UV wavelength, 254 nm; t_{R} of (+)-*N*-(2-methoxyphenyl)-1-(4-methoxyphenyl)ethylamine 8.9 min.; t_{R} of (-)-*N*-(2-methoxyphenyl)-1-(4-methoxyphenyl)ethylamine 10.0 min.); specific rotation [α]²⁵_{D} -27.1° (c 1.01, CHCl₃).

### [Example 18]

### Synthesis of (-)-N-(2-methoxyphenyl)-1-(3-chlorophenyl)ethylamine by asymmetric hydrogenation of N-[1-(3-chlorophenyl)ethylidene]-2-methoxyaniline

A glass autoclave was charged with RuBr₂[(*S,S*)-xylskewphos][(*S,S*)-dpen] (0.51 mg, 0.5 µmol) and KOC(CH₃)₃ (5.7 mg, 51 µmol) and flushed with argon. 0.34 mL of toluene and N-[1-(3-chlorophenyl)ethylidene]-2-methoxyaniline (0.34 mL, 1.5 mmol) were added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 98% ee (-)-*N*-(2-methoxyphenyl)-1-(3-chlorophenyl)ethylamine was formed in a yield of >99%.

### [Example 19]

### Synthesis of (-)-N-(2-methoxyphenyl)-1-(4-chlorophenyl)ethylamine by asymmetric hydrogenation of N-[1-(4-chlorophenyl)ethylidene]-2-methoxyaniline

A glass autoclave was charged with RuBr₂[(*S,S*)-xylskewphos][(*S,S*)-dpen] (0.51 mg, 0.5 µmol), *N*-[1-(4-chlorophenyl)ethylidene]-2-methoxyaniline (390 mg, 1.5 mmol), and KOC(CH₃)₃ (5.7 mg, 51 µmol) and flushed with argon. 0.34 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 98% ee (-)-*N*-(2-methoxyphenyl)-1-(4-chlorophenyl)ethylamine was formed in a yield of 99%. The spectral data of the amine compound obtained were as follows. ¹H NMR (400 MHz, CDCl₃) δ 1.52 (d, J = 6.9 Hz, 3H, CH₃), 3.88 (s, 3H, OCH₃), 4.43 (q, 6.9 Hz, 1H, CHNH), 4.60 (br, 1H, NH), 6.26 (dd, J = 1.8 Hz, 7.8 Hz, 1H, aromatic H), 6.59-6.77 (m, 3H, aromatic H), 7.25-7.31 (m, 4H, aromatic H); ¹³C NMR (100 MHz, CDCl₃) δ 25.2, 52.8, 55.4, 109.3, 111.0, 116.6, 121.1, 127.2, 128.7, 132.3, 136.9, 144.0, 146.5; HPLC (column, CHIRALCEL OD-H; solvent, hexane/2-propanol = 99/1; flow rate, 1.0 mL/min; temperature, 30°C; UV wavelength, 254 nm; t_{R} of (+)-*N*-(2-methoxyphenyl)-1-(4-chlorophenyl)ethylamine 7.4 min.; t_{R} of (-)-*N*-(2-methoxyphenyl)-1-(4-chlorophenyl)ethylamine 9.9 min.); specific rotation [α]²⁵_{D} - 34.2° (c 1.01, CHCl₃).

### [Example 20]

### Synthesis of (-)-N-(2-methoxyphenyl)-1-(2-fluorophenyl)ethylamine by asymmetric hydrogenation of N-[1-(2-fluorophenyl)ethylidene]-2-methoxyaniline

A glass autoclave was charged with RuBr₂[(*S,S*)-xylskewphos][(*S,S*)-dpen] (1.0 mg, 1 µmol) and KOC(CH₃)₃ (5.7 mg, 51 µmol) and flushed with argon. 0.36 mL of toluene and *N*-[1-(2-fluorophenyl)ethylidene]-2-methoxyaniline (0.32 mL, 1.5 mmol) were added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 91% ee (-)-*N*-(2-methoxyphenyl)-1-(2-fluorophenyl)ethylamine was formed in a yield of >99%. The spectral data of the amine compound obtained were as follows. ¹H NMR (400 MHz, CDCl₃) δ 1.56 (d, J = 6.9 Hz, 3H, CH₃), 3.89 (s, 3H, OCH₃), 4.61 (br, 1H, NH), 4.81 (q, 6.9 Hz, 1H, CHNH), 6.33 (dd, J = 1.8 Hz, 7.8 Hz, 1H, aromatic H), 6.59-6.78 (m, 3H, aromatic H), 7.01-7.37 (m, 4H, aromatic H); ¹³C NMR (100 MHz, CDCl₃) δ 23.4, 46.9 (d, J = 2.9 Hz), 55.4, 109.3, 110.8, 115.3 (d, J = 22.0 Hz), 116.5, 121.2, 124.3 (d, J = 3.8 Hz), 127.0 (d, J = 4.8 Hz), 128.1 (d, J = 7.7 Hz), 131.9 (d, J = 13.4 Hz), 136.7, 146.6, 160.5 (d, J = 244.4 Hz); HPLC (column, CHIRALCEL OD-H; solvent, hexane/2-propanol = 99/1; flow rate, 0.5 mL/min; temperature, 30°C; UV wavelength, 254 nm; t_{R} of (+)-*N*-(2-methoxyphenyl)-1-(2-fluorophenyl)ethylamine 11.7 min.; t_{R} of (-)-N-(2-methoxyphenyl)-1-(2-fluorophenyl)ethylamine 14.1 min.); specific rotation [α]²⁵_{D} -26.0° (c 1.02, CHCl₃).

### [Example 21]

### Synthesis of (-)-N-(2-methoxyphenyl)-1-(4-fluorophenyl)ethylamine by asymmetric hydrogenation of N-[1-(4-fluorophenyl)ethylidene]-2-methoxyaniline

A glass autoclave was charged with RuBr₂[(*S,S*)-xylskewphos][(*S,S*)-dpen] (0.51 mg, 0.5 µmol), *N*-[1-(4-fluorophenyl)ethylidene]-2-methoxyaniline (365 mg, 1.5 mmol), and KOC(CH₃)₃ (5.7 mg, 51 µmol) and flushed with argon. 0.35 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 98% ee (-)-*N*-(2-methoxyphenyl)-1-(4-fluorophenyl)ethylamine was formed in a yield of >99%. The spectral data of the amine compound obtained were as follows. ¹H NMR (400 MHz, CDCl₃) δ 1.52 (d, J = 6.9 Hz, 3H, CH₃), 3.88 (s, 3H, OCH₃), 4.44 (q, 6.9 Hz, 1H, CHNH), 4.59 (br, 1H, NH), 6.29 (dd, J = 1.4 Hz, 7.8 Hz, 1H, aromatic H), 6.59-6.77 (m, 3H, aromatic H), 6.96-7.01 (m, 2H, aromatic H), 7.25-7.34 (m, 2H, aromatic H); ¹³C NMR (100 MHz, CDCl₃) δ 25.3, 52.7, 55.4, 109.3, 111.0, 115.3 (d, J = 22.0 Hz), 116.5, 121.1, 127.3 (d, J = 7.7 Hz), 137.0, 141.1 (d, J = 2.9 Hz), 146.5, 161.7 (d, J = 244.4 Hz); HPLC (column, CHIRALCEL OD-H; solvent, hexane/2-propanol = 99/1; flow rate, 0.5 mL/min; temperature, 30°C; UV wavelength, 254 nm; t_{R} of (+)-N-(2-methoxyphenyl)-1-(4-fluorophenyl)ethylamine 18.5 min.; t_{R} of (-)-*N*-(2-methoxyphenyl)-1-(4-fluorophenyl)ethylamine 26.6 min.); specific rotation [α]²⁵_{D} -55.1° (c 1.03, CHCl₃).

### [Example 22]

### Synthesis of (-)-N-(2-methoxyphenyl)-1-(4-trifluoromethylphenyl)ethylamine by asymmetric hydrogenation of N-[1-(4-trifluoromethylphenyl)ethylidene]-2-methoxyaniline

A glass autoclave was charged with RuBr₂[(*S,S*)-xylskewphos][(*S,S*)-dpen] (0.51 mg, 0.5 µmol), *N*-[1-(4-trifluoromethylphenyl)ethylidene]-2-methoxyaniline (440 mg, 1.5 mmol), and KOC(CH₃)₃ (8.8 mg, 79 µmol) and flushed with argon. 0.65 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 97% ee (-)-N-(2-methoxyphenyl)-1-(4-trifluoromethylphenyl)ethylamine was formed in a yield of >99%. The spectral data of the amine compound obtained were as follows. ¹H NMR (400 MHz, CDCl₃) δ 1.55 (d, J = 6.4 Hz, 3H, CH₃), 3.90 (s, 3H, OCH₃), 4.51 (q, 6.4 Hz, 1H, CHNH), 4.65 (br, 1H, NH), 6.23 (dd, J = 1.8 Hz, 7.8 Hz, 1H, aromatic H), 6.61-6.79 (m, 3H, aromatic H), 7.48 (d, J = 8.2 Hz, 2H, aromatic H), 7.56 (d, J = 8.2 Hz, 2H, aromatic H); ¹³C NMR (100 MHz, CDCl₃) δ 25.1, 53.1, 55.4, 109.3, 110.9, 116.8, 121.1, 124.2 (q, J = 272.2 Hz), 125.6 (q, J = 3.8 Hz), 126.2, 129.1 (q, J = 31.6 Hz), 136.7, 146.6, 149.7; HPLC (column, CHIRALCEL OD-H; solvent, hexane/2-propanol = 99/1; flow rate, 1.0 mL/min; temperature, 30°C; UV wavelength, 254 nm; t_{R} of (+)-*N*-(2-methoxyphenyl)-1-(4-trifluoromethylphenyl)ethylamine 7.8 min.; t_{R} of (-)-N-(2-methoxyphenyl)-1-(4-trifluoromethylphenyl)ethylamine 11.5 min.); specific rotation [α]²⁵_{D} -40.8° (c 1.01, CHCl₃).

### [Example 23]

### Synthesis of (R)-N-(2-methoxyphenyl)-1-(2-naphthyl)ethylamine by asymmetric hydrogenation of N-[1-(2-naphthyl)ethylidene]-2-methoxyaniline

A glass autoclave was charged with RuBr₂[(*S,S*)-xylskewphos][(*S,S*)-dpen] (0.51 mg, 0.5 µmol), *N*-[1-(2-naphthyl)ethylidene]-2-methoxyaniline (413 mg, 1.5 mmol), and KOC(CH₃)₃ (8.8 mg, 79 µmol) and flushed with argon. 0.67 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 98% ee (*R*)-*N*-(2-methoxyphenyl)-1-(2-naphthyl)ethylamine was formed in a yield of >99%. The spectral data of the amine compound obtained were as follows. ¹H NMR (400 MHz, CDCl₃) δ 1.62 (d, J = 6.9 Hz, 3H, CH₃), 3.91 (s, 3H, OCH₃), 4.61 (q, 6.9 Hz, 1H, CHNH), 4.71 (br, 1H, NH), 6.37 (dd, J = 1.4 Hz, 7.8 Hz, 1H, aromatic H), 6.57-6.78 (m, 3H, aromatic H), 7.40-7.52 (m, 3H, aromatic H), 7.78-7.81 (m, 4H, aromatic H); ¹³C NMR (100 MHz, CDCl₃) δ 25.2, 53.6, 55.4, 109.2, 111.2, 116.4, 121.2, 124.2, 124.4, 125.4, 125.9, 127.6, 127.8, 128.4, 132.7, 133.6, 137.2, 143.0, 146.6; HPLC (column, CHIRALCEL OD-H; solvent, hexane/2-propanol = 99/1; flow rate, 0.5 mL/min; temperature, 30°C; UV wavelength, 254 nm; t_{R} of (*S*)-*N*-(2-methoxyphenyl)-1-(2-naphthyl)ethylamine 17.2 min.; t_{R} of (*R*)-*N*-(2-methoxyphenyl)-1-(2-naphthyl)ethylamine 21.2 min.); specific rotation [α]²⁵_{D} -40.8° (c 1.00, CHCl₃); literature value, [α]²⁵_{D} -76.8° (c 1.04, CHCl₃, 99% ee (R)) J. Am. Chem. Soc. 2009, 131, 8358.

### [Example 24]

### Synthesis of (-)-N-(2-methoxyphenyl)-1-(3-pyridyl)ethylamine by asymmetric hydrogenation of N-[1-(3-pyridyl)ethylidene]-2-methoxyaniline

A glass autoclave was charged with RuBr₂[(*S,S*)-xylskewphos][(*S,S*)-dpen] (2.1 mg, 2 µmol) and KOC(CH₃)₃ (3.8 mg, 34 µmol) and flushed with argon. 0.24 mL of toluene and *N*-[1-(3-pyridyl)ethylidene]-2-methoxyaniline (0.21 mL, 1 mmol) were added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 98% ee (-)-*N*-(2-methoxyphenyl)-1-(3-pyridyl)ethylamine was formed in a yield of >99%. The spectral data of the amine compound obtained were as follows. ¹H NMR (400 MHz, CDCl₃) δ 1.58 (d, J = 6.9 Hz, 3H, CH₃), 3.89 (s, 3H, OCH₃), 4.52 (br. m, 1H, CHNH), 4.62 (br. d, J = 1.4 Hz, 1H, NH), 6.28-6.79 (m, 4H, aromatic H), 7.20-7.24 (m, 1H, aromatic H), 7.66-7.69 (m, 1H, aromatic H), 8.48 (dd, J = 1.8 Hz, 5.0 Hz, 1H, aromatic H), 8.63 (d, J = 2.3 Hz, 1H, aromatic H); ¹³C NMR (100 MHz, CDCl₃) δ 25.0, 51.2, 55.4, 109.4, 111.0, 116.9, 121.1, 123.6, 133.4, 136.6, 140.7, 146.6, 148.3, 148.4; HPLC (column, CHIRALCEL OJ-H; solvent, hexane/2-propanol = 95/5; flow rate, 1.0 mL/min; temperature, 30°C; UV wavelength, 254 nm; t_{R} of (-)-N-(2-methoxyphenyl)-1-(3-pyridyl)ethylamine 15.0 min.; t_{R} of (+)-N-(2-methoxyphenyl)-1-(3-pyridyl)ethylamine 21.1 min.); specific rotation [α]²⁵_{D} -59.8° (c 0.31, CHCl₃).

### [Example 25]

### Synthesis of (-)-N-(2-methoxyphenyl)-1-(4-pyridyl)ethylamine by asymmetric hydrogenation of N-[1-(4-pyridyl)ethylidene]-2-methoxyaniline

A glass autoclave was charged with RuBr₂[(*S,S*)-xylskewphos][(*S,S*)-dpen] (2.1 mg, 2 µmol), *N*-[1-(4-pyridyl)ethylidene]-2-methoxyaniline (226 mg, 1 mmol), and KOC(CH₃)₃ (3.8 mg, 34 µmol) and flushed with argon. 0.24 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 95% ee (-)-N-(2-methoxyphenyl)-1-(4-pyridyl)ethylamine was formed in a yield of 95%. The spectral data of the amine compound obtained were as follows. ¹H NMR (400 MHz, CDCl₃) δ 1.53 (d, J = 6.4 Hz, 3H, CH₃), 3.90 (s, 3H, OCH₃), 4.44 (dd, J = 6.4 Hz, 13.3 Hz, 1H, CHNH), 4.63 (br. s, 1H, NH), 6.20 (m, 1H, aromatic H), 6.62-6.80 (m, 3H, aromatic H), 7.30 (d, J = 5.5 Hz, 2H, aromatic H), 8.53 (d, J = 5.5 Hz, 2H, aromatic H); ¹³C NMR (100 MHz, CDCl₃) δ 24.5, 52.6, 55.4, 109.4, 110.9, 117.0, 121.1, 121.2, 136.5, 146.5, 149.9, 154.8; HPLC (column, CHIRALCEL OD-H; solvent, hexane/2-propanol = 90/10; flow rate, 0.5 mL/min; temperature, 30°C; UV wavelength, 254 nm; t_{R} of (+)-N-(2-methoxyphenyl)-1-(4-pyridyl)ethylamine 21.8 min.; t_{R} of (-)-N-(2-methoxyphenyl)-1-(3-pyridyl)ethylamine 36.4 min.); specific rotation [α]²⁵_{D} -31.1° (c 1.01, CHCl₃).

### [Example 26]

### Synthesis of (R)-N-(2-methoxyphenyl)-1-phenylpropylamine by asymmetric hydrogenation of N-(1-phenylpropylidene)-2-methoxyaniline

A glass autoclave was charged with RuBr₂[(*S,S*)-xylskewphos][(*S,S*)-dpen] (0.51 mg, 0.5 µmol), N-(1-phenylpropylidene)-2-methoxyaniline (359 mg, 1.5 mmol), and KOC(CH₃)₃ (5.7 mg, 51 µmol) and flushed with argon. 0.35 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 96% ee (*R*)-*N*-(2-methoxyphenyl)-1-phenylpropylamine was formed in a yield of >99%. The spectral data of the amine compound obtained were as follows. ¹H NMR (400 MHz, CDCl₃) δ 0.97 (t, J = 7.3 Hz, 3H, CH₃), 1.81-1.92 (m, 2H, CH₂), 3.88 (s, 3H, OCH₃), 4.21 (t, J = 6.9 Hz, ¹H, CHNH), 4.69 (br, 1H, NH), 6.33 (dd, J = 1.4 Hz, 7.8 Hz, 1H, aromatic H), 6.56-6.76 (m, 3H, aromatic H), 7.18-7.35 (m, 5H, aromatic H); ¹³C NMR (100 MHz, CDCl₃) δ 10.9, 31.7, 55.5, 59.6, 109.2, 110.8, 116.1, 121.1, 126.5, 126.8, 128.4, 137.5, 144.2, 146.6; HPLC (column, CHIRALCEL OD-H; solvent, hexane/2-propanol = 99/1; flow rate, 0.5 mL/min; temperature, 30°C; UV wavelength, 254 nm; t_{R} of (*S*)-*N*-(2-methoxyphenyl)-1-phenylpropylamine 11.7 min.; t_{R} of (R)-*N*-(2-methoxyphenyl)-1-phenylpropylamine 13.9 min.); specific rotation [α]²⁵_{D} -12.7° (c 1.02, CHCl₃); literature value, [α]²⁵_{D} +7.7° (c 7.0, CHCl₃, 95% ee (*S*)) J. Am. Chem. Soc. 2001, 123, 984.

### [Example 27]

### Synthesis of (-)-N-(2-methoxyphenyl)-1-[3-[N'-(2-methoxyphenyl)aminoethyl]phenyl]ethylamine by asymmetric hydrogenation of N-[1-[3-[N'-(2-methoxyphenyl)iminoethyl]phenyl]ethylidene]-2-methoxyaniline

A glass autoclave was charged with RuBr₂[(*S,S*)-xylskewphos][(*S,S*)-dpen] (0.51 mg, 0.5 µmol), *N*-[1-[3-[*N'*-(2-methoxyphenyl)iminoethyl]phenyl]ethylidene]-2-methoxyaniline (559 mg, 1.5 mmol), and KOC(CH₃)₃ (15.2 mg, 136 µmol) and flushed with argon. 1.3 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 92% de and >99% ee (-)-N-(2-methoxyphenyl)-1-[3-[*N'*-(2-methoxyphenyl)aminoethyl]phenyl]ethylamine was formed in a yield of >99%. The spectral data of the amine compound obtained were as follows. ¹H NMR (400 MHz, CDCl₃) δ 1.52 (d, J = 6.4 Hz, 6H, CH₃ x2), 3.88 (s, 6H, OCH₃ x2), 4.42 (q, J = 6.4 Hz, 2H, CHNH ×2), 4.59 (br. s, 2H, NH x2), 6.25-6.27 (m, 2H, aromatic H), 6.58-6.78 (m, 6H, aromatic H), 7.19-7.34 (m, 4H, aromatic H); ¹³C NMR (100 MHz, CDCl₃) δ 25.1, 53.5, 55.4, 109.2, 111.2, 116.3, 121.2, 123.7, 124.2, 128.9, 137.3, 145.7, 146.5; HPLC (column, CHIRALCEL OD-H; solvent, hexane/2-propanol = 98/2; flow rate, 1.0 mL/min; temperature, 30°C; UV wavelength, 254 nm; t_{R} of (+)-N-(2-methoxyphenyl)-1-[3-[*N'*-(2-methoxyphenyl)aminoethyl]phenyl]ethylamine 11.0 min.; t_{R} of (-)-*N*-(2-methoxyphenyl)-1-[3-[*N'*-(2-methoxyphenyl)aminoethyl]phenyl]ethylamine 16.5 min.); specific rotation [α]²⁵_{D} -67.0° (c 1.04, CHCl₃).

### [Example 28]

### Synthesis of (-)-N-(2-methoxyphenyl)-1-[3-[N'-(2-methoxyphenyl)aminoethyl]phenyl]ethylamine by asymmetric hydrogenation of N-[1-[3-[N'-(2-methoxyphenyl)iminoethyl]phenyl]ethylidene]-2-methoxyaniline

A glass autoclave was charged with RuBr₂[(*S,S*)-skewphos][(S,S)-dpen] (0.91 mg, 1 µmol), *N*-[1-[3-[*N'*-(2-methoxyphenyl)iminoethyl]phenyl]ethylidene]-2-methoxyaniline (373 mg, 1 mmol), and KOC(CH₃)₃ (10.1 mg, 90 µmol) and flushed with argon. 0.86 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 93% de and >99% ee (-)-N-(2-methoxyphenyl)-1-[3-[*N'*-(2-methoxyphenyl)aminoethyl]phenyl]ethylamine was formed in a yield of >99%.

### [Example 29]

### Synthesis of (+)-N-(3-bromophenyl)-1-(4-chloro-3-methylphenyl)ethylamine by asymmetric hydrogenation of N-[1-(4-chloro-3-methylphenyl)ethylidene]-3-bromoaniline

A glass autoclave was charged with RuBr₂[(*S,S*)-xylskewphos][(S,S)-dpen] (0.51 mg, 0.5 µmol), *N*-[1-(4-chloro-3-methylphenyl)ethylidene]-3-bromoaniline (403 mg, 1.25 mmol), and KOC(CH₃)₃ (4.8 mg, 43 µmol) and flushed with argon. 0.2 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 96% ee (+)-*N*-(3-bromophenyl)-1-(4-chloro-3-methylphenyl)ethylamine was formed in a yield of >99%. The spectral data of the amine compound obtained were as follows. ¹H NMR (400 MHz, CDCl₃) δ 1.48 (d, J = 6.8 Hz, 3H, CHCH₃), 2.35 (s, 3H, PhCH₃), 4.09 (br. s, 1H, NH), 4.38 (q, J = 6.8 Hz, 1H, CHNH), 6.37-7.27 (m, 7H, aromatic H); ¹³C NMR (100 MHz, CDCl₃) δ 20.2, 24.9, 52.9, 106.2, 111.9, 116.1, 120.3, 123.1, 124.5, 128.3, 129.3, 130.4, 132.8, 136.3, 143.0; HPLC (column, CHIRALCEL OJ-H; solvent, hexane/2-propanol = 95/5; flow rate, 1.0 mL/min; temperature, 30°C; UV wavelength, 254 nm; t_{R} of (+)-N-(3-bromophenyl)-1-(4-chloro-3-methylphenyl)ethylamine 19.0 min.; t_{R} of (-)-N-(3-bromophenyl)-1-(4-chloro-3-methylphenyl)ethylamine 38.3 min.); specific rotation [α]²⁵_{D} +6.4° (c 1.02, CHCl₃).

### [Example 30]

### Synthesis of (+)-N-[4-(N'-Boc-N'-methylamino)phenyl]-1-(4-chlorophenyl)ethylamine by asymmetric hydrogenation of N-[1-(4-chlorophenyl)ethylidene]-4-(N'-Boc-N'-methylamino)aniline

A glass autoclave was charged with RuBr₂[(*S,S*)-xylskewphos][(*S,S*)-dpen] (0.51 mg, 0.5 µmol), *N*-[1-(4-chlorophenyl)ethylidene]-4-(N'-Boc-N'-methylamino)aniline (538 mg, 1.5 mmol), and KOC(CH₃)₃ (8.8 mg, 79 µmol) and flushed with argon. 0.56 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 93% ee (+)-*N*-[4-(*N'*-Boc-*N'-*methylamino)phenyl]-1-(4-chlorophenyl)ethylamine was formed in a yield of 99%. The spectral data of the amine compound obtained were as follows. ¹H NMR (400 MHz, CDCl₃) δ 1.41 (br. s, 9H, C(CH₃)₃), 1.48 (d, J = 6.9 Hz, 3H, CHCH₃), 3.14 (s, 3H, NCH₃), 3.98 (br, 1H, NH), 4.41 (q, J = 6.9 Hz, 1H, CHNH), 6.40 (d, J = 8.7 Hz, 2H, aromatic H), 6.92 (br. d, J = 7.3 Hz, 2H, aromatic H), 7.29 (br, 4H, aromatic H); ¹³C NMR (100 MHz, CDCl₃) δ 25.1, 28.3, 37.7, 53.2, 79.7, 113.2, 126.7, 127.2, 128.8, 132.4, 134.1, 143.7, 144.8, 155.3; HPLC (column, CHIRALCEL OD-H; solvent, hexane/2-propanol = 98/2; flow rate, 1.0 mL/min; temperature, 30°C; UV wavelength, 254 nm; t_{R} of (+)-*N*-[4-(*N'*-Boc-*N'-*methylamino)phenyl]-1-(4-chlorophenyl)ethylamine 25.6 min.; t_{R} of (-)-*N*-[4-(*N'*-Boc-*N'*-methylamino)phenyl]-1-(4-chlorophenyl)ethylamine 31.8 min.); specific rotation [α]²⁵_{D} +23.2° (c 1.01, CHCl₃).

### [Example 31]

### Synthesis of (+)-N-[4-(N'-Boc-N'-methylamino)phenyl]-1-(4-chlorophenyl)ethylamine by asymmetric hydrogenation of N-[1-(4-chlorophenyl)ethylidene]-4-(N'-Boc-N'-methylamino)aniline

A reaction was carried out under the same conditions as those of Example 30 except that the ruthenium complex was changed to RuBr₂[(*S,S)*-skewphos] [(*S,S*)-dpen], thus giving 95% ee (+)-*N-*[4-(N'-Boc-*N'*-methylamino)phenyl]-1-(4-chlorophenyl)ethylamine in a yield of >99%.

### [Example 32]

### Synthesis of 1-[N-(3,4,5-trimethoxyphenyl)]aminoindane by asymmetric hydrogenation of N-(1-indanylidene)-3,4,5-trimethoxyaniline

A glass autoclave was charged with RuBr₂[(*S,S*)-xylskewphos][(*S,S*)-4-CF₃dpen] (9.30 mg, 8.0 µmol), *N*-(1-indanylidene)-3,4,5-trimethoxyaniline (595 mg, 2.0 mmol), and KOC(CH₃)₃ (11.8 mg, 105 µmol) and flushed with argon. 0.94 mL of toluene was added thereto, and the autoclave was degassed and then flushed with hydrogen. It was charged with hydrogen until the pressure reached 10 atm, and a reaction was started by stirring in a water bath at 40°C. 21 hours later the pressure was returned to normal pressure. From ¹H NMR and HPLC of the product, it was found that 87.4% ee 1-[N-(3,4,5-trimethoxyphenyl)]aminoindane was formed in a yield of >99%. The spectral data of the amine compound obtained were as follows. ¹H NMR (400 MHz, CDCl₃) δ 1.95 (m, 1H, CHH) , 2.58 (m, 1H, CHH), 2.90 (m, 1H, CHH), 3.02 (m, 1H, CHH), 3.78 (s, 3H, OCH₃), 3.82 (s, 6H, OCH₃ x2), 4.98 (t, J = 6.9 Hz, 1H, CHNH), 5.98 (s, 2H, aromatic H), 7.19-7.40 (m, 4H, aromatic H); ¹³C NMR (100 MHz, CDCl₃) δ 30.2, 33.7, 56.0, 59.4, 61.1, 91.3, 91.3, 124.3, 124.9, 126.6, 128.0, 143.6, 154.0; HPLC (column, CHIRALCEL OD-H; solvent, hexane/2-propanol = 95/5; flow rate, 1.0 mL/min; temperature, 30°C; UV wavelength, 254 nm; t_{R} of 1-[N-(3,4,5-trimethoxyphenyl)]aminoindane 21.6 min., 27.5 min.).

### [Example 33]

### Synthesis of 1-[N-(3,4,5-trimethoxyphenyl)]aminoindane by asymmetric hydrogenation of N-(1-indanylidene)-3,4,5-trimethoxyaniline

A reaction was carried out under the same conditions as those of Example 32 except that the Ru complex was changed to RuBr₂[(*S,S*)-modskewphos][(*S,S*)-4-CF₃dpen] and S/C was changed to 500, thus giving 85.7% ee 1-[N-(3,4,5-trimethoxyphenyl)]aminoindane in a yield of 57%.

### [Examples 34 to 38]

### Synthesis of 1-[N-(3,4-dimethoxyphenyl)]aminoindane by asymmetric hydrogenation of N-(1-indanylidene)-3,4-dimethoxyaniline

1-[*N*-(3,4-Dimethoxyphenyl)]aminoindane was synthesized by carrying out a reaction using various Ru complexes. The results are summarized in Table 4.

**[Table 4]**

| | | | |
|---|---|---|---|
| 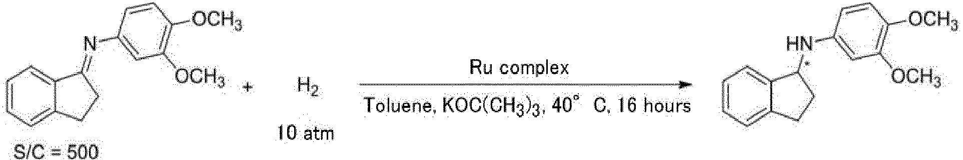 | | | |
| Substrate concentration 1.43 M | | | |
| Base concentration 75 mM | | | |

| Example | Ru complex | Yield(%) | ee(%) |
|---|---|---|---|
| 34 | RuBr₂[(*S,S*)-xylskewphos][(*S,S*)-dpen] | 79 | 43 |
| 35 | RuBr₂[(*S,S*)-xylskewphos][(*S,S*)-4-CF₃dpen] | 55 | 60 |
| 36 | RuBr₂[(*S,S*)-xylskewphos][(*S,S*)-4-Fdpen] | 11 | 54 |
| 37 | RuBr₂[(S,S)-xylskewphos][(S,S)-4-MeOdpen] | 86 | 51 |
| 38 | RuBr₂[(*S,S*)-xylskewphos][(*S,S*)-3-MeOdpen] | 97 | 48 |

### [Example 39]

### Synthesis of (-)-N-(2-methoxyphenyl)-4-methyl-3-penten-2-ylamine by asymmetric hydrogenation of N-(4-methyl-3-penten-2-ylidene)-2-methoxyaniline

A glass autoclave was charged with RuBr₂[(*S,S*)-xylskewphos][(*S,S*)-dpen] (0.51 mg, 0.5 µmol) and KOC(CH₃)₃ (5.7 mg, 51 µmol) and flushed with argon. N-(4-Methyl-3-penten-2-ylidene)-2-methoxyaniline (0.31 mL, 1.5 mmol) and 0.37 mL of toluene were added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 85% ee (-)-N-(2-methoxyphenyl)-4-methyl-3-penten-2-ylamine was formed in a yield of 78%. The spectral data of the amine compound obtained were as follows. ¹H NMR (400 MHz, CDCl₃) δ 1.27 (d, J = 6.9 Hz, 3H, CHCH₃), 1.69 (d, J = 1.4 Hz, 3H, C(CH₃)₂), 1.74 (d, J = 1.4 Hz, 3H, C(CH₃)₂), 3.83 (s, 3H, OCH₃), 4.13 (m, 1H, CHNH), 4.13 (br, 1H, NH), 5.08 (d, J = 8.7 Hz, 1H, CH=C), 6.55-6.86 (m, 4H, aromatic H); ¹³C NMR (100 MHz, CDCl₃) δ 18.1, 22.0, 25.6, 46.9, 55.3, 109.3, 110.6, 116.1, 121.2, 129.8, 132.4, 137.6, 146.7; HPLC (column, CHIRALCEL OJ-H; solvent, hexane/2-propanol = 99/1; flow rate, 1.0 mL/min; temperature, 30°C; UV wavelength, 254 nm; t_{R} of (-)-N-(2-methoxyphenyl)-4-methyl-3-penten-2-ylamine 6.8 min.; t_{R} of (+)-N-(2-methoxyphenyl)-4-methyl-3-penten-2-ylamine 9.2 min.); specific rotation [α]²⁵_{D} -29.2° (c 1.03, CHCl₃).

### [Example 40]

### Synthesis of RuH(BH₄)[(S,S)-xylskewphos][(S,S)-dpen] complex

By the same method as in J. Am. Chem. Soc. 2002, 124, 6508-6509, a RuH(BH₄)[(*S,S*)-xylskewphos][(*S,S*)-dpen] complex was synthesized. That is, a Schlenk tube was charged with RuBr₂[(*S,S*)-xylskewphos][(*S,S*)-dpen] (308 mg, 0.30 mmol) and NaBH₄ (28.4 mg, 0.75 mmol) and flushed with argon. 12 mL of a degassed mixed solvent of benzene/ethanol (1:1) was added thereto, and stirring was carried out under an atmosphere of argon in an oil bath at 65°C for 5 min. and then at room temperature for 1 hour. The solvent was removed by distillation to dryness under reduced pressure, 18 mL of benzene was added, stirring was carried out, and filtration was carried out using a glass filter under a flow of argon to thus remove an insoluble substance. The filtrate was concentrated to dryness under reduced pressure, thus giving 300 mg of RuH(BH₄)[(*S,S*)-xylskewphos][(*S,S*)-dpen] as a brown powder. ³¹P NMR (162 MHz, C₆D₅CD₃) δ 67.8 (br. d, J = 52.3 Hz), 70.3 (d, J = 48.0 Hz), 72.7 (dd, J = 8.7 Hz, 43.6 Hz), 75.4 (d, J = 48.0 Hz).

### [Examples 41 and 42]

A reaction was carried under the same conditions as those of Example 1 except that the ruthenium complex, S/C, and the reaction time were changed, thus synthesizing (R)-N-(2-methoxyphenyl)-1-phenylethylamine. The results are summarized in Table 5.

**[Table 5]**

| | | | | |
|---|---|---|---|---|
| 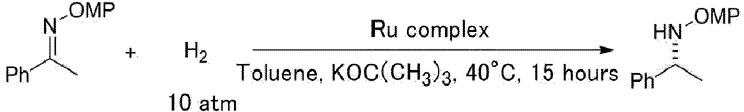 | | | | |
| Substrate concentration 2.20 M | | | | |
| Base concentration 75 mM | | | | |

| Example | Ru complex | S/C | Yield (%) | ee (%) |
|---|---|---|---|---|
| 41 | RuH(BH₄)[(*S,S*)-xylskewphos][(*S,S*)-dpen] | 4000 | >99 | 99 |
| 42 | RuBr₂[(*S,S*)-xylskewphos][(*S*)-pen] | 500 | 96 | 97 |

### [Example 43]

### Synthesis of (R)-N-benzyl-1-phenylethylamine by asymmetric hydrogenation of N-(1-phenylethylidene)benzylamine

A glass autoclave was charged with RuH(BH₄) [(*S,S*)-xylskewphos][(*S,S*)-dpen] (0.88 mg, 1.0 µmol), *N*-(1-phenylethylidene)benzylamine (209 mg, 1.0 mmol), and NaOC(CH₃)₃ (10.1 mg, 105 µmol) and flushed with argon. 0.26 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR and HPLC of the product, it was found that 90% ee (*R*)-*N*-benzyl-1-phenylethylamine was formed in a yield of 99%. The spectral data of the amine compound obtained were as follows. ¹H NMR (400 MHz, CDCl₃) δ 1.37 (d, J = 6.4 Hz, 3H, CHCH₃), 3.59 (d, J = 12.8 Hz, 1H, CHHPh), 3.66 (d, J = 13.3 Hz, 1H, CHHPh), 3.81 (q, J = 6.4 Hz, 1H, CHNH), 7.14-7.40 (m, 10H, aromatic H); ¹³C NMR (100 MHz, CDCl₃) δ 24.5, 51.6, 57.5, 126.7, 126.8, 126.9, 128.1, 128.3, 128.4, 140.6, 145.6; HPLC (column, CHIRALCEL OD-H; solvent, hexane/2-propanol/NHEt₂ = 99.5/0.5/0.1; flow rate, 0.5 mL/min; temperature, 30°C; UV wavelength, 254 nm; t_{R} of (*R*)-*N*-benzyl-1-phenylethylamine 24.1 min.; t_{R} of (*S*)-*N*-benzyl-1-phenylethylamine 30.1 min.); specific rotation [α]²⁵_{D} +43.7° (c 1.03, CHCl₃); literature value, [α]²⁰_{D} +23.7° (c 1.35, C₂H₅OH, (R)) J. Org. Chem. 1990, 55, 1086.

### [Comparative Example 1]

### Synthesis of (S)-N-(4-methoxyphenyl)-1-phenylethylamine by asymmetric hydrogenation of N-(1-phenylethylidene)-4-methoxyaniline

A glass autoclave was charged with RuCl₂[(*R*)-binap][(*R,R*)-dpen] (5.03 mg, 5.0 µmol), *N*-(1-phenylethylidene)-4-methoxyaniline (225 mg, 1.0 mmol), and KOC(CH₃)₃ (11.2 mg, 100 µmol) and flushed with argon. 1.0 mL of toluene was added thereto, and the autoclave was degassed and then flushed with hydrogen. It was charged with hydrogen until the pressure reached 10 atm, and a reaction was started by stirring in a water bath at 50°C. 15 hours later the pressure was returned to normal pressure. From ¹H NMR and HPLC of the product, it was found that 44% ee (*S*)-*N*-(4-methoxyphenyl)-1-phenylethylamine was formed in a yield of 60%.

### [Comparative Example 2]

### Asymmetric hydrogenation reaction of N-(1-phenylethylidene)-2-methoxyaniline

A glass autoclave was charged with RuCl₂[(*R,R*)-xyldiop][(*S,S*)-dpen] (1.00 mg, 1.0 µmol), *N*-(1-phenylethylidene)-2-methoxyaniline (225 mg, 1.0 mmol), and KOC(CH₃)₃ (3.8 mg, 34 µmol) and flushed with argon. 0.25 mL of toluene was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR it was found that the reaction had not proceeded at all.

### [Comparative Example 3]

A reaction was carried out under the same conditions as those of Comparative Example 2 except that the Ru complex was changed to RuCl₂[(*R*)-1-[(*S*)-2-(dicyclohexylphosphino)ferrocenyl]ethyldiphenylphosphine][(*R,R*)-dpen], but the reaction did not proceed at all.

### [Comparative Example 4]

### Asymmetric hydrogenation reaction of N-(1-phenylethylidene)-2-methoxyaniline

A glass autoclave was charged with RuBr₂[(*S,S*)-xylskewphos][(S,S)-dpen] (1.03 mg, 1 µmol), *N*-(1-phenylethylidene)-2-methoxyaniline (225 mg, 1 mmol), and KOC(CH₃)₃ (5.6 mg, 50 µmol) and flushed with argon. 2-Propanol 1 mL was added thereto, and the autoclave was degassed and then flushed with hydrogen. It was charged with hydrogen until the pressure reached 10 atm, and a reaction was started by stirring in a water bath at 40°C. 15 hours later the pressure was returned to normal pressure. From ¹H NMR it was found that the reaction had not proceeded at all.

### [Industrial Applicability]

The optically active amine compound obtained by the method for producing an optically active amine compound of the present invention does not require a complicated and expensive purification process, and the like for optical resolution, and may be used directly in the synthesis of for example a pharmaceutical compound, an agrochemical compound, an intermediate therefor, and the like.

## Claims

**1.** A method for producing an optically active amine compound represented by Formula (5) 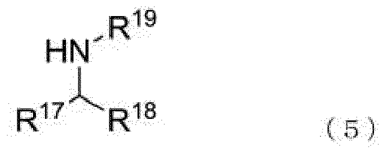 (R¹⁷ and R¹⁸ are independently selected from the group consisting of optionally substituted alkyl, optionally substituted alkenyl, optionally substituted alkynyl, optionally substituted cycloalkyl, optionally substituted cycloalkenyl, optionally substituted aryl, optionally substituted heteroaryl, optionally substituted aralkyl, and optionally substituted heteroaralkyl,
R¹⁹ is hydrogen, optionally substituted alkyl, optionally substituted alkenyl, optionally substituted alkynyl, optionally substituted cycloalkyl, optionally substituted cycloalkenyl, optionally substituted aryl, optionally substituted heteroaryl, optionally substituted aralkyl, or optionally substituted heteroaralkyl, and
two of R¹⁷, R¹⁸, and R¹⁹ may together form an optionally substituted ring), in which a ruthenium complex represented by Formula (1)
RuXYAB (1)
(X and Y are each independently hydrogen or an anionic group, A is a diphosphine compound represented by Formula (2), 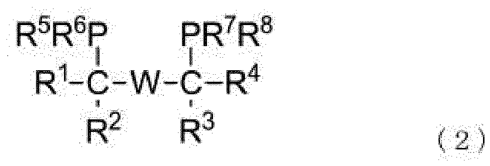 (R¹, R², R³, and R⁴ are each independently hydrogen or an optionally substituted chain-form or cyclic hydrocarbon group having 1 to 20 carbons,
R⁵, R⁶, R⁷, and R⁸ are each independently an optionally substituted hydrocarbon group having 1 to 30 carbons, and
W is an optionally substituted hydrocarbon group having 1 or 2 carbons, or a single bond), and
B is a diamine compound represented by Formula (3) 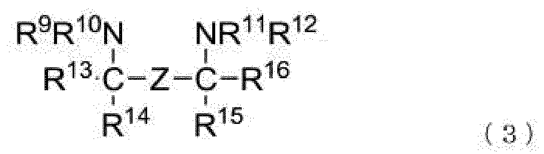 (R⁹, R¹⁰, R¹¹, and R¹² are each independently hydrogen or an optionally substituted chain-form or cyclic hydrocarbon group having 1 to 30 carbons, wherein at least one of R⁹, R¹⁰, R¹¹, and
R¹² is hydrogen,
R¹³, R¹⁴, R¹⁵, and R¹⁶ are each independently hydrogen or an optionally substituted chain-form or cyclic hydrocarbon group having 1 to 30 carbons, and
Z is an optionally substituted chain-form or cyclic hydrocarbon group having 1 to 10 carbons, or a single bond), each of the ligands of ruthenium being able to coordinate in any way),
an imine compound represented by Formula (4) 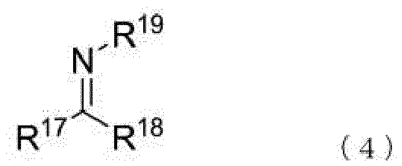 (R¹⁷, R¹⁸, and R¹⁹ are as defined above), and
one or more bases selected from the group consisting of an alkali metal or alkaline earth metal salt, a quaternary ammonium salt,
and a tertiary amine are mixed in an aprotic solvent or without a solvent under pressurized hydrogen to thus hydrogenate the imine compound.

**2.**
A method for producing an optically active amine compound represented by Formula (5) 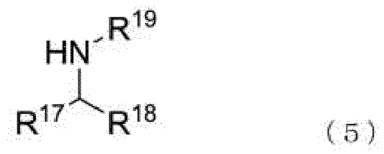 (R¹⁷, R¹⁸, and R¹⁹ are as defined in Claim 1),
in which a ruthenium complex represented by Formula (6)
RuXYALₙ (6)
(L is an organic ligand, n is any number between 0 and 2, and X, Y, and A are as defined in Claim 1),
a diamine compound represented by Formula (3) 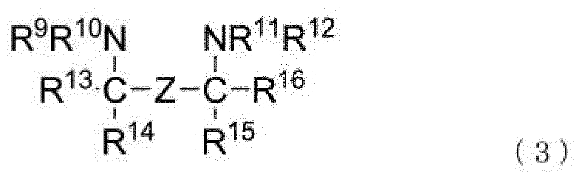 (R⁹, R¹⁰, R¹¹, R¹², R¹³, R¹⁴, R¹⁵, and R¹⁶ are as defined in Claim 1), an imine compound represented by Formula (4) 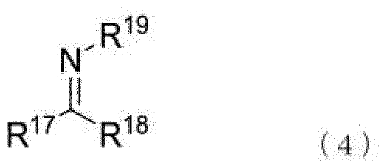 (R¹⁷, R¹⁸, and R¹⁹ are as defined in Claim 1), and one or more bases selected from the group consisting of an alkali metal or alkaline earth metal salt, a quaternary ammonium salt,
and a tertiary amine are mixed in an aprotic solvent or without a solvent under pressurized hydrogen to thus hydrogenate the imine compound.

**3.** The method according to Claim 1 or 2, wherein in Formula (1) or (6), A is: 2,4-bis(diphenylphosphino)pentane,: 2,4-bis(di-4-tolylphosphino)pentane,: 2,4-bis(di-3,5-xylylphosphino)pentane, or: 2,4-bis[bis(3,5-dimethyl-4-methoxyphenyl)phosphino]pentane.

**4.** The method according to Claim 1, wherein in Formula (3), R⁹, R¹⁰, R¹¹, R¹², R¹³, and R¹⁵ are hydrogen; R¹⁴ is hydrogen, a phenyl group, a 4-trifluoromethylphenyl group, a 4-fluorophenyl group, a 4-methoxyphenyl group, or a 3-methoxyphenyl group; R¹⁶ is a phenyl group, a 4-trifluoromethylphenyl group, a 4-fluorophenyl group, a 4-methoxyphenyl group, or a 3-methoxyphenyl group; and Z is a single bond.

**5.** The method according to Claim 1, wherein the aprotic solvent is one or more selected from the group consisting of toluene, benzene, tetrahydrofuran, and *tert*-butyl methyl ether.

**6.** The method according to 1, wherein in Formula (4), R¹⁷ is optionally substituted alkenyl, optionally substituted alkynyl, optionally substituted cycloalkenyl, optionally substituted aryl, or optionally substituted heteroaryl; R¹⁸ is different from R¹⁷ and is optionally substituted alkyl, optionally substituted cycloalkyl, optionally substituted alkenyl, optionally substituted cycloalkenyl, or optionally substituted aryl; R¹⁷ and R¹⁸ may together form an optionally substituted ring; and R¹⁹ is optionally substituted alkyl, optionally substituted alkenyl, optionally substituted alkynyl, optionally substituted aryl, optionally substituted heteroaryl, or optionally substituted aralkyl.

**7.** The method according to Claim 1, wherein in Formula (4), R¹⁷ is optionally substituted alkenyl, optionally substituted aryl, or optionally substituted heteroaryl; R¹⁸ is optionally substituted alkyl or optionally substituted cycloalkyl; R¹⁷ and R¹⁸ may together form an optionally substituted ring; and R¹⁹ is an optionally substituted alkyl, optionally substituted aryl, or optionally substituted aralkyl.
